# EUROPEAN PATENT SPECIFICATION

(11) **EP 1 830 956 B1**
(45) Date of publication and mention of the grant of the patent: **30.11.2022**
(21) Application number: 05820631.9
(22) Date of filing: 23.12.2005
(51) Int. Cl.: B01J 29/04, B01J 29/70, B01J 29/18, B01J 29/40

(54) **METHOD FOR THE PREPARATION OF CATALYTIC MATERIALS**
VERFAHREN ZUR HERSTELLUNG VON KATALYTISCH WIRKSAMEN MATERIALIEN
PROCÉDÉ DE PRÉPARATION DE MATÉRIAUX CATALYTIQUES

(30) Priority: 28.12.2004 FI 20041675; 28.12.2004 US 639314 P
(43) Date of publication of application: 12.09.2007
(60) Divisional application: 22173046.8
(73) Proprietor: Neste Oyj, 02150 Espoo (FI)
(72) Inventor: KUMAR, Narendra, 20750 Turku (FI); TIITTA, Marja, 06150 Porvoo (FI); SALMI, Tapio, 20720 Turku (FI); ÖSTERHOLM, Heidi, 06100 Porvoo (FI)
(74) Representative: Espatent Oy
(86) International application number: PCT/FI2005/050484
(87) International publication number: WO 2006/070073

(56) References cited:
- US-A- 5 051 385
- US-A1- 2001 031 241
- US-B1- 6 492 014
- KLOETSTRA K R ET AL: "OVERGROWTH OF MESOPOROUS MCM-41 ON FAUJASITE", MICROPOROUS MATERIALS, ELSEVIER SCIENCE BV, AMSTERDAM, NL, vol. 6, no. 5/06, 1 July 1996 (1996-07-01) , pages 287-293, XP000911747, ISSN: 0927-6513, DOI: 10.1016/0927-6513(96)00036-3
- WANPING GUO ET AL: "Synthesis and characterization of composite molecular sieves comprising zeolite Beta with MCM-41 structures", JOURNAL OF MATERIALS CHEMISTRY, vol. 11, no. 7, 1 January 2001 (2001-01-01), pages 1886-1890, XP55028173, ISSN: 0959-9428, DOI: 10.1039/b009903l
- GUO W ET AL: "Characterization of Beta/MCM-41 composite molecular sieve compared with the mechanical mixture", MICROPOROUS AND MESOPOROUS MATERIALS, ELSEVIER SCIENCE PUBLISHING, NEW YORK, US, vol. 44-45, 6 April 2001 (2001-04-06), pages 427-434, XP004247175, ISSN: 1387-1811, DOI: 10.1016/S1387-1811(01)00217-7
- CUNMAN ZHANG ET AL.: 'Synthesis and characterization of composite molecular sieves with mesoporous and microporous structure from ZSM-5 zeolites by heat treatment' MICROPOROUS AND MESOPOROUS MATERIALS vol. 62, 2003, pages 157 - 163, XP004444307
- HUANG L ET AL: "INVESTIGATION OF SYNTHESIZING MCM-41/ZSM-5 COMPOSITES", JOURNAL OF PHYSICAL CHEMISTRY. B, MATERIALS, SURFACES, INTERFACES AND BIOPHYSICAL, WASHINGTON, DC, US, vol. 104, no. 13, 6 April 2000 (2000-04-06), pages 2817-2823, XP000992110, ISSN: 1089-5647, DOI: 10.1021/JP990861Y
- FRANK D. MANGO: "The light hydrocarbons in petroleum: a critical review", ORGANIC GEOCHEMISTRY, vol. 26, no. 7-8, 1 April 1997 (1997-04-01), pages 417-440, XP055633458, AMSTERDAM, NL ISSN: 0146-6380, DOI: 10.1016/S0146-6380(97)00031-4

## Description

### Field of the Invention

The invention is related to a method for the preparation of catalytic materials which are mesoporous molecular sieves embedded with a zeolite. Said catalytic materials are suitable for applications in the field of hydrocarbon processing.

### State of the Art

Mesoporous molecular sieves as catalytic materials have attracted the attention of scientists because of their unique properties, such as large uniform pores having a very high surface area, the size of which can be varied from 2 to 50 nm. However, mesoporous molecular sieves known in the art are often thermally and hydrothermally not very stable, the pore walls are amorphous and they have mild acidic properties. Further more, during the regeneration of the spent catalyst after hydrocarbon processing, the mesoporous molecular sieve structure may collapse.

Crystalline materials having pore size in the microporous area (d < 2 nm) are used as catalysts and as carriers of catalysts on an industrial scale. Zeolites are well-known examples of such materials. Zeolites are widely used because of their special properties, such as large surface area, high capacity of adsorption and possibility to regulate adsorption capacity. It is possible to create active sites in the zeolite structure, build up active sites and to regulate the strength and amount of the acid sites. The pore size of zeolites is typically in the range of 0.4 - 1.2 nm and both the thermal and chemical stability of zeolites are high. However, the ability of zeolites to process molecules having larger molecular size than the pore size of the zeolites is limited and further, zeolites are deactivated relatively rapidly in several reactions.

US 5,198,203 discloses a family of ordered, mesoporous molecular sieves, designated as M41S and developed in the beginning of 90's. M41S is a group of mesoporous molecular sieve materials formed in an aqueous solution with silica and alumina precursors with CᵢH₂ᵢ(CH₃)N⁺- cations (i > 7) at hydrothermal conditions. The most well known members of this group are hexagonal MCM-41, cubic MCM-48 and plate-like structure MCM-50. The pore size of the mesoporous molecular sieve can be regulated between 2 and 10 nm and the composition may contain pure silica or metallosilica (e.g. Al-, V- and Ti-substituted silica). The mesoporous molecular sieve materials of the M41S group are amorphous by nature and their pore system is ordered.

A synthetic composition of a material comprising ultra-large pore crystalline phase is disclosed in US 5,246,689 and US 5,334,368. This material is inorganic, porous and non-layered having pore dimensions between 1.3 and 20 nm. The pore size distribution within a single phase is to some extent regular. At least one peak in the X-ray diffraction pattern at d-spacing is greater than 1.8 nm.

EP 0 748 652 discloses a group of mesoporous materials (MSA) having a narrow pore size distribution. This material was amorphous and totally disordered. The BET surface area of the material was in the range of 672 - 837 m²/g.

Synthetically produced mesoporous materials are not acidic or their acidity is limited. The amount of acid sites in mesoporous materials has been increased by incorporation of aluminium in the silica structure of the mesoporous material. The strength of the acidity of the mesoporous materials described above is, however, less than the strength of the acidity of the zeolites.

Various methods for the manufacture of mesoporous materials are known in the art. Attempts have been made to increase thermal and hydrothermal stability and acidity of mesoporous molecular sieves, for example by introducing catalytically active species in the mesoporous structures. In principal, the methods of synthesis comprise preparation of a silicon source solution with an organic agent or agents, adjusting the pH of the solution to a value where precipitation occurs, followed by recovering and calcination of the precipitate. An aluminium source is added to the solution in any step prior to the starting of the synthesis at elevated temperature. Several surfactants and templates (organic agents), compositions, solvents and reaction conditions have been suggested.

US 5,942,208 describes a method for the preparation of mesoporous material having improved hydrothermal stability when compared to MCM-41. Various salts were used in the method and the pH of the solution was adjusted with mild acids.

EP 0 795 517 provides a method for the synthesis of mesoporous materials wherein a mixture of a silicon source and organic template containing fluorine was used.

US 5,942,208 describes the preparation of a mesoporous molecular sieve having thermal and hydrothermal stability superior to the ones of traditional mesoporous molecular sieves. The material could be boiled in water for 12 hours without essential changes in the structure.

An alternative approach for the manufacture of stable and active mesoporous materials is to introduce zeolites into the walls of the mesopores. US 09/764,686 discloses the synthesis of mesoporous materials using Y-zeolite seed, MFI zeolite seed and beta-zeolite seed.

CN 1349929 teaches the preparation of MSA-3 and MAS-8 using L-zeolite precursor solutions.

Kloetstra et al, Micropor. Mesopor. Mater. 6 (1996) 287 have reported *in situ* formation of faujasite and MCM-41. Their approach was based on sequential synthesis of zeolites and MCM-41.

Guo et al. J. Mater. Chem., 11, (2001) 1886 describe synthesis and characterization of zeolite Beta/MCM-41 composites with various crystallinities of Beta through a two-step crystallization process involving the combination of low crystallinity zeolite Beta synthesis gel with cetyltrimethylammonium bromide surfactant solution.

Guo et al. Microporous and Mesoporous Materials 44-45 (2001) 427 describe the characterization of Beta /MCM-41 composite molecular sieve and compare it with a mechanical mixture of Beta and MCM-41 for their structure, acidity and catalytic activity.

Cunman Zhang et al. Microporous and Mesoporous Materials 62 (2003) 157 describe composite molecular sieves from ZSM-5 zeolites by heat treatment.

Karlsson et al,. Micropor. Mesopor Mater. 27 (1999) 181 disclose the use of mixed template approach for the simultaneous synthesis of zeolite/MCM-41 phases.

Huang et al. J. Phys. Chem. B 104 (2000) 21817 teaches the preparation of MCM-41/ZSM-5 composites by a dual templating method through a process of two-step crystallization.

US 2001/031241 A1 discloses an acidic silicoaluminate solid that has a microporous zeolitic phase and an organized mesoporosity, and a process for the preparation thereof.

The materials may be mixtures of two or more phases, or loosely bonded zeolite and mesoporous material in the case the synthesis approach is to grow and deposit MCM-41 over zeolite, or zeolite seeds may be added to the gel.

Two different types of templates have been used in the synthesis of mesoporous materials. The reproducibility of such manufacturing methods may be difficult. Furthermore, in the absence of chemical interaction between the zeolite and mesoporous molecular sieve, thermal and hydrothermal stability of the resulting materials is likely to be low.

According to the state of the art, mesoporous molecular sieves have a wide range of applications in catalysis as active phases or as supports. Several hydrocarbon conversion reactions are acid catalysed. Based on their acid catalysed function, zeolites are know to be active in double-bond and skeletal isomerization of olefins, isomerization of paraffins, cracking, dimerization of olefins, oligomerization of olefins, ring opening of naphthenes, alkylation, transalkylation of aromatics, aromatization etc. Bifunctional catalyst having metal or metal-oxide or sulfide phases are applicable in reactions such as reforming, isomerization of paraffins, hydrocracking, catalytic dewaxing, dehydrosulfurization, dehydrooxygenation, dehydronitrogenation and several hydrogenation reactions. The major drawbacks in the use of the zeolites are their relative high ability for deactivation and limited capacity of handling of bulky molecules.

Based on the above it can be seen that there exists a need for thermally and hydrothermally stable catalytic materials based on mesoporous molecular sieves and for a method for the preparation of such thermally and hydrothermally stable catalytic materials. It is also obvious that there is a need for a catalyst having zeolite type active site but also high accessibility of the active sites for reactants, short diffusion path length of reactants and products limiting secondary reactions and coking.

### Object of the Invention

An object of the invention is a method for the manufacture of said catalytic material having mesoporous molecular sieve embedded with a zeolite structure.

### Summary of the Invention

Without wishing to be restricted by the following explanations and theoretical considerations regarding the novel synthesis of the catalytic material having mesoporous molecular sieve embedded with a zeolite structure, which is particularly suitable for hydrocarbon conversion reactions, the essential features of the invention are discussed as follows.

The invention relates to a method for the preparation of the mesoporous molecular sieve embedded with a zeolite whereby the synthesis is facilitated and reproducible and the product exhibits high catalytic activity.

The catalytic material having mesoporous molecular sieve embedded with zeolite structure is suitable for hydrocarbon conversion reactions and particularly for processing of high molecular weight hydrocarbons. This catalytic material can be used as a component of a catalyst in cracking, hydrocracking, ring opening, hydrogenation of aromatics and especially multiaromatics, dimerization of olefins, oligomerization, isomerization of olefins and paraffins, alkylation of aromatics, etherification, hydrodesulphurization and reforming, or as such or with modifications known from the state of art.

### Detailed Description of the Invention

It has now been found that the problems relating to zeolite catalysts and mesoporous catalysts according to the state of the art can be avoided or at least significantly decreased by the catalytic material produced according to the invention, which is a mesoporous molecular sieve embedded with zeolite, having high mechanical, thermal and hydrothermal stability. The mesoporous molecular sieve embedded with a zeolite produced according to the invention is thermally stable at temperatures of at least 900 ⁰C in the presence of air.

The mesoporous molecular sieves embedded with zeolites are mechanically, thermally and hydrothermally stable. The materials are very well reproducible as can be seen in the examples, and they exhibit superior properties in several hydrocarbon conversion reactions. The group of the mesoporous molecular sieves embedded with zeolites are named mesoporous materials (MM). Mesoporous means here materials having pores of 2 - 15 nm and their pore system is regular.

The mesoporous molecular sieve embedded with a zeolite comprises a mesoporous molecular sieve selected from M41S group, which is defined on page 2 and comprises mesoporous materials with ordered pore system. Preferably the mesoporous molecular sieve is selected from mesoporous alumino-silicates known as the MCM-41 group.

The mesoporous molecular sieve is embedded with a zeolite selected from medium pore zeolites, which are 10-member ring zeolites like MFI, MTT, TON, AEF, MWW and FER structures, and large pore zeolites, which are 12-member ring zeolites like BEA, FAU and MOR structures. Examples of said zeolite groups are ZSM-5, ZSM-23, ZSM-22, SAPO-11, MCM-22, ferrierite, beta, Y-and X-zeolites and mordenite. Preferably the zeolite is MFI, MTT, AEF, MWW, MOR or BEA zeolite.

The catalytic material contains 0.01 - 10 wt-% of aluminium (Al).

A catalyst, which is particularly suitable for industrial and commercial use, comprises the mesoporous molecular sieve embedded with a zeolite and also a carrier selected from alumina, silica, clay and any other carrier according to the state of the art, and combinations thereof. Preferably the carrier comprises alumina or silica. The amount of the carrier varies between 10 - 90 wt-%, calculated on the total weight of the catalyst.

The group of catalytic materials having a mesoporous molecular sieve embedded with a zeolite structure exhibits high specific surface area (BET) in the range of 1400 - 500 m²/g, preferably 1200 - 600 m²/g.

The X-ray powder diffraction pattern of the catalytic material demonstrates the mesoporous molecular sieve and zeolite structures. The unit cell dimension of the zeolite varies with amount of Al in the catalytic material. The unit cell size decreases with the amount of Al, from 1.982 nm in a catalytic material containing 0.2 wt% of Al to 1.972 nm in a catalytic material containing 3.9 wt% of Al, when the zeolite type was MFI (the code of the material is MM5). The change in the unit cell size is opposite to the changes observed in zeolites in general.

The unit cell sizes were 1.428 nm and 1.430 nm, when the zeolite type was BEA (the code of the material is MMBE), the unit cell sizes were 1.406 nm and 1.436 nm, when the zeolite type was MWW (the code of the material is MMMW22) and the unit cell sizes were 1.800 nm and 1.806 nm, when the zeolite type was MOR (the code of the material is MMMO).

The d₁₀₀ spacing in the mesoporous molecular sieve MCM-41 decreases with increasing zeolite content. The d₁₀₀ varies from 4.4 nm to 3.8 nm in MM5 and the d₁₀₀ varies from 4.1 nm to 4.0 nm in MMBE and MMMO, and from 4.0 to 4.2 in MMMW

The unit cell dimension and the d₁₀₀ values are the same in pure zeolite and MCM-41 phases as in their mechanical mixtures.

The changes in the d₁₀₀ spacing and the unit cell dimension are a clear evidence of a true chemical bonding between the mesoporous molecular sieve and the embedded zeolite in the catalytic material.

The characteristic features of the catalytic material, the mesoporous molecular sieve embedded with a zeolite, were measured by X-ray powder diffraction, scanning electron microscopy, transmission electron microscopy, specific surface area measurement using nitrogen absorption (BET) and acidity measurements using ammonia-TPD and pyridine-FTIR.

The total number of acid sites can be measured by the capacity of the catalytic material to bind strong base molecules, such as ammonia or pyridine. The total acidity was measured by ammonia-temperature programmed desorption (TPD) and Bronsted and Lewis acidity by pyridine-infrared spectroscopy (FTIR).

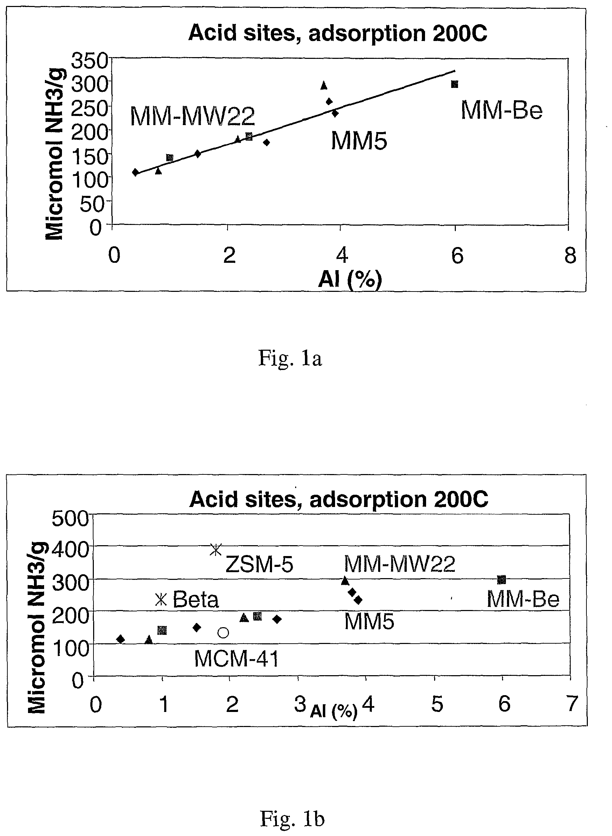

The acidity of the catalytic material can be tailored by the amount of Al introduced in the structure and modifying the aluminium (Al) content in the zeolite, MCM-41 and MM phases. In Figures 1a and 1b the correlation between the acidity and aluminium content in the catalytic materials prepared according to the invention is presented. Figure 1a shows the linearity of total acidity as a function of Al-content in the various MM catalytic materials and Figure 1 b shows how the zeolitic and the MCM-41 catalytic materials deviate in total acidity from the MM catalytic materials. The zeolites exhibit larger amount of acid sites as a function of Al content than MM5, MMBE and MMMW samples, MCM-41 is less acidic with similar aluminium content.

Since there are no international standard methods available for acidity determination, the methods used here are described below.

Acidity determination was performed by NH₃-TPD. The total acidity of catalytic materials was measured by temperature-programmed desorption of ammonia (NH₃-TPD) using an Altamira AMI-100 instrument. Sample size was 40 mg. The total acidity was measured by desorption of NH₃ as a function of temperature. The acidity of the samples was calculated from the amount of NH₃ adsorbed at 200 °C and desorbed between 100 °C and 500 °C. The NH₃-TPD instrument was equipped with a thermal conductivity detector (TCD) manufactured by company Gow Mac. A ramp rate of 20 °C/min was applied and the temperature was linearly raised to 500 °C where it was held for 30 min. The quantification was made using pulses of known volume of 10 % NH₃ in He.

Acidity determination was determined also by pyridine-FTIR. The acidity of samples was measured by infrared spectroscopy (ATI Mattson FTIR) by using pyridine (≥ 99.5 %, a.r.) as a probe molecule for qualitative and quantitative determination of both Brønsted and Lewis acid sites. The samples were pressed into thin self-supported wafers (10-12 mg/cm²). Pyridine was first adsorbed for 30 min at 100 °C and then desorbed by evacuation at different temperatures (250, 300, and 450 °C) to obtain a distribution of acid site strengths. All spectra were recorded at 100 °C with a spectral resolution equal to 2 cm⁻¹. Spectral bands at 1545 cm⁻¹ and 1450 cm⁻¹, respectively, were used for identifying Brønsted (BAS) and Lewis acid sites (LAS). The amounts of BAS and LAS were calculated from the intensities of corresponding spectral bands by using the molar extinction coefficients.

The acid sites are situated on the surface of the catalytic material. The total surface area and pore volume were evaluated using N₂-adsorption and desorption. The average mesopore surface area and mesopore diameter were evaluated from the N₂-desorption utilizing the BJH (Barrer-Joyner-Halenda) equation. The pore diameter has a size-limiting effect both on reactants and products. The size of micropores depends on the structure of the zeolite. Pores with a diameter less than 2 nm are defined as micropores and pores with a diameter between 2 and 50 nm are defined as mesopores according to IUPAC.

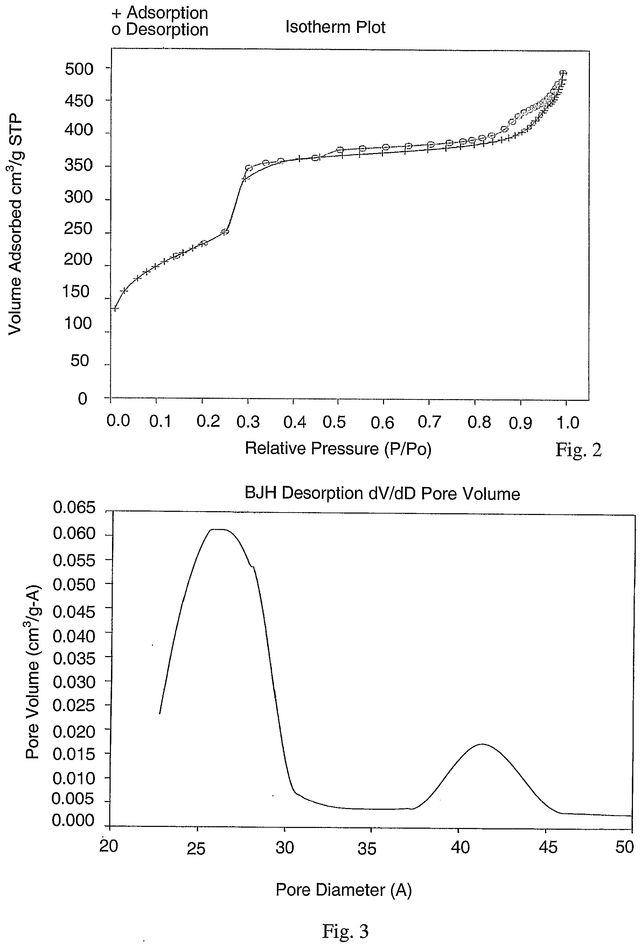

The N₂-absorption desorption isotherms of MMBE are shown in Figure 2. The mesopore diameter remains similar, 2.4 - 2.7 nm, in the embedded material compared to 2.6 nm in the mesoporous molecular sieve.

BJH desorption illustrating mesopore diameter distribution in MMBE is shown in attached Figure 3.

The surface area and total pore volume decrease when the zeolite is embedded in the mesoporous molecular sieve as can be seen from table 1 below, presenting surface area, pore volume and pore diameter values for MM5, MMBE and MMMW, and for comparison MCM-41, MFI and BEA data are enclosed.

**Table 1 . Surface area and porosity**

| **Sample** | **BET area (m²/g)** | **BJH mesopore area (m²/g)** | **Total pore volume (cm³/g)** | **BJH pore diameter (nm)** |
|---|---|---|---|---|
| Na-MCM-41-20 | 949 | 947 | 0.829 | 2.6 |
| Na-MM5-2ZS | 896 | 1145 | 0.814 | 2.5 |
| Na-MM5-4ZS | 820 | 1009 | 0.713 | 2.6 |
| Na-MM5-4ZS-2Al | 867 | 1069 | 0.794 | 2.5 |
| Na-MM5-4ZS-2Al25 | 733 | 599 | 0.656 | 2.4 |
| MFI ZSM-5 | 360 | 100 | 0.351 | 20^{∗} |
| Na-MMBE-4B | 879 | 884 | 0.692 | 2.7 |
| Na-MMBE-4B-2Al | 844 | 859 | 0.742 | 2.7 |
| Na-MMBE-4B-2Al35 | 793 | 684 | 0.835 | 2.6 |
| BEA | 585 | 85 | 0.254 | None |
| H-MM-MW22 | 854 | 838 | 0.755 | 2.5 |
| H-MM-MW22-2Al | 808 | 772 | 0.703 | 2.5 |
| H-MM-MW22-2Al-35 | 728 | 639 | 0.834 | 2.5 |

| | | | | |
|---|---|---|---|---|
| ^{∗} = interparticle void size | | | | |

The zeolite is identified by X-ray diffraction (XRD). From the XRD patterns the unit cell dimensions of the zeolite as well as of the MCM-41 phase can be measured when suitable internal standards are used. α-Al₂O₃ or TiO₂ (rutile) was used as the internal standard.

The unit cell size of MFI was measured by ASTM D 3942-97 method, using α-Al₂O₃ as internal standard.

The unit cell of BEA was measured by a modified ASTM D 3942-97 method, using TiO₂ as internal standard and the [302] reflection at 22°2 theta.

The unit cell of MWW was measured by a modified ASTM D 3942-97 method, using α-Al₂O₃ as internal standard and the [100] reflection at 7.2°2 theta.

The unit cell size of MMMO was estimated from the peak positions without internal standard. The estimation was ao=2 ^{∗} d_{[100]}/√3.

The unit cell size of the mesoporous molecular sieve (MCM-41) was estimated by a method described by J.S. Becker et.al., J. Am. Chem. Soc. 114 (1992) 10834.

The unit cell size of zeolites corresponds to the amount of Al incorporated in the zeolite framework. The Al atom is larger than the Si atom, thus the unit cell size typically increases with increasing amount of Al in most zeolites. On the contrary, in the embedded zeolite the unit cell dimension decreases with increasing amount of Al in the MM5 catalytic material, as can be seen from table 2 (MFI, BEA, MWW and MOR ao values). The change in unit cell dimension (UCD) of MCM-41 does not correlate with the amount of Al, it rather decreases with increasing intensity of the MFI phase. The changes in unit cell dimensions are clear evidences of true chemical bonding between the mesoporous molecular sieve and the embedded zeolite.

Aluminium content and unit cell dimensions of MCM-41, as well as of MFI, BEA, MWW and MOR zeolites embedded in mesoporous molecular sieve (MCM-41) are presented in the following table 2.

**Table 2. Aluminium content and unit cell dimensions**

| **Sample** | **Al (%)** | **MCM-41 ao (nm)** | **MFI, BEA MWW and MOR a0 (nm)** |
|---|---|---|---|
| MCM-41 | 2.5 | 3.5 | - |
| Na-MM5-2ZS | 0.2 | 4.4 | 1.982 |
| Na-MM5-4ZS | 0.4 | 4.2 | 1.981 |
| Na-MM5-4ZS-2Al | 1.5 | 4.3 | 1.979 |
| Na-MM5-4ZS-2Al35 | 3.9 | 3.8 | 1.972 |
| MFI ZSM-5 | 1.4 | - | 1.978 |
| Na-MM-BE-4B | 1.0 | 4.0 | 1.428 |
| Na-MM-BE-4B-2Al | 2.4 | 4.1 | 1.428 |
| Na-MM-BE-4ZS-2Al35 | 6.0 | 4.1 | 1.430 |
| Na-MM-MW22-2Al | 2.2 | 4.2 | 1.426 |
| H-MM-MW22 | 0.8 | 4.1 | 1.406 |
| H-MM-MW22-2Al | 2.2 | 4.1 | 1.436 |
| H-MM-MW22-2Al-35 | 3.7 | 4.0 | 1.427 |
| MCM-22 | 2.4 | - | 1.405 |
| Na-MM-MO | 1.0 | 4.1 | 1.800 |
| Na-MM-MO-2Al | 4.6 | 4.1 | 1.800 |
| H-MM-MO | 1.0 | 4.0 | 1.806 |
| Pt/H-MM-MO | 1.1 | 4.1 | 1.806 |

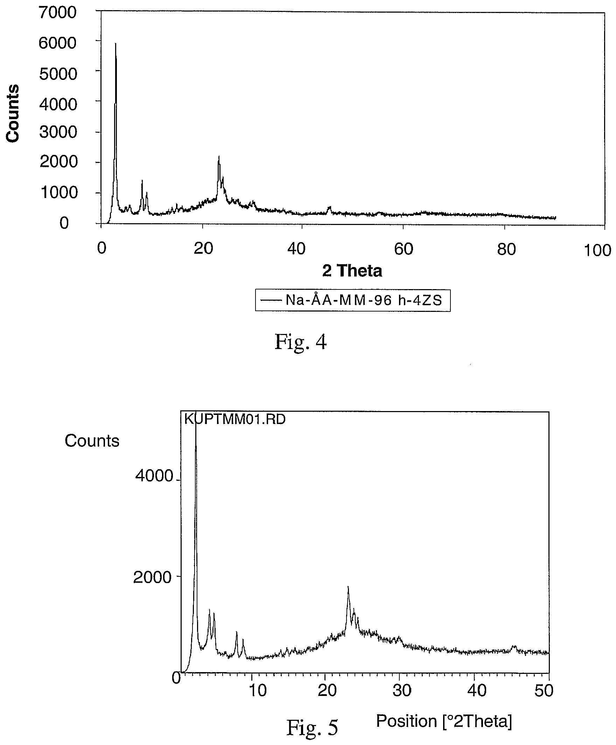

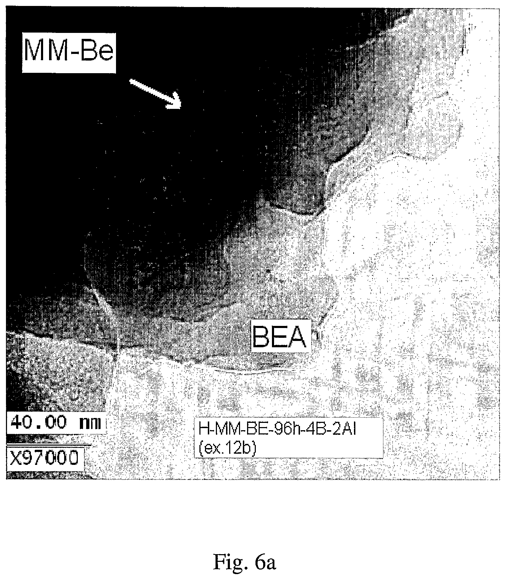

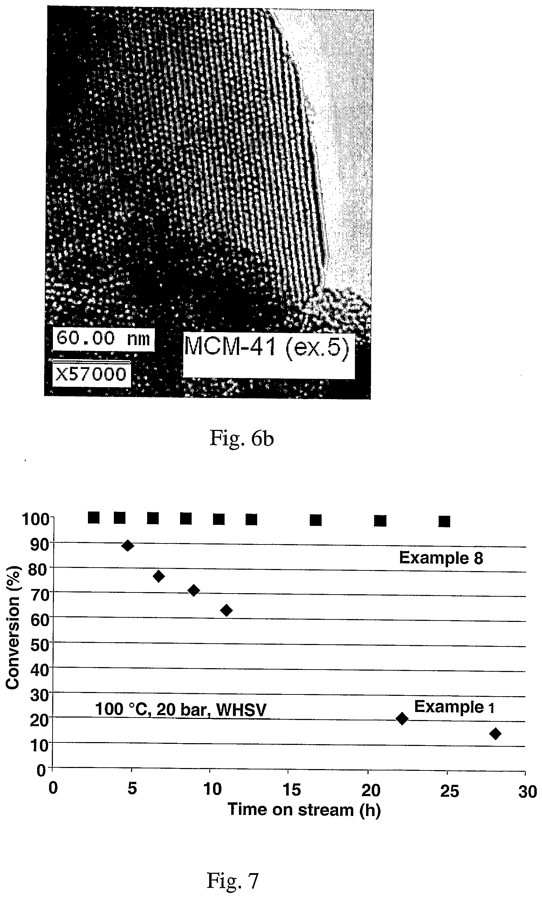

Thermal stability of the catalytic material obtained by the method according to the invention was tested by exposing the embedded material to a temperature of 1000°C in air. The XRD diffraction pattern of calcined MM5 is shown in Figure 4. After thermal treatment at 1000 °C the same pattern is obtained, as shown in Figure 5. This observation provides evidence that this catalytic material obtained by the method according to the invention is thermally stable to a temperature of at least 1000°C. The nanostructure of the catalytic materials obtained by the method according to the invention was studied by high-resolution transmission electron microscopy using (HRTEM) (Philips CM-200FEG transmission electron microscope with point resolution of 0.24 nm). The composition was measured with EDS (NORAN Voyager energy dispersive X-ray spectrometer). In Fig. 6a a HRTEM picture of the mesoporous material embedded with beta zeolite obtained by the method according to the invention is illustrated. For comparison, in Fig 6b, HRTEM picture of an ordered MCM-41 material is shown.

The method for the manufacture of the mesoporous molecular sieve embedded with a zeolite is described in more detail in the following.

The method for the manufacture of a catalytic material which catalytic material is a zeolite embedded within a mesoporous molecular sieve, wherein the zeolite is selected from MFI, MTT, TON, AEF, MWW, FER, BEA, FAU, MOR zeolites:
a) preparing of zeolite nuclei from a silicon source and an aluminium source and structure directing agent (template R), and optionally removing the template with a step calcination procedure;
b) preparing of mesoporous molecular sieve gel mixture from a silicon source, an optional aluminium source, and surfactant (S);
c) introducing the zeolite nuclei, prepared in step a) to the mesoporous molecular sieve gel mixture obtained in step b), and homogenising and dispersing in the molecular sieve gel the zeolite nuclei, and introducing an additional aluminium source to the obtained mixture;
d) performing gel ripening of the mixture of step c) under stirring;
e) carrying out hydrothermal synthesis of the mixture of step c) by maintaining the mixture under sufficient conditions including a temperature of from about 100 °C to about 200 °C under static or dynamic mode of stirring until crystals are formed;
f) recovering the crystals;
g) washing of the solid product;
h) drying of the solid product, and
i) removing the surfactant (S) partly or totally with a step calcination procedure and optionally the template (R) if it was not removed in step a), whereby a mesoporous molecular sieve embedded with a zeolite catalyst is obtained.

In step a) the zeolite nuclei are prepared from a silicon source and an aluminium source and structure directing agent (template R). The silicon source is selected from silicon oxides, preferably from colloidal silica, solid silica and fumed silica.

The aluminium source is selected from aluminium sulphate (Al₂(SO₄)₃.18H₂O), hydrated aluminium hydroxides, aluminates, aluminium isoproxide and alumina.

A suitable template is selected in order to obtain the desired zeolite structure. Examples of typically used templates are alkyl ammonium hydroxides, alkyl ammonium halogenides, alkyl amine hydroxide and alkyl amine halogenides like tetrapropylammonium bromide, tetramethylammonium hydroxide, tetramethylammonium bromide, tetraethylammonium bromide, tetraethylammonium hydroxide, piperidine, pyrrolidine, octylamine, ethylenediamine, 1,6-diaminohexane and hexamethyleneimine.

The temperature in step a) is between 40 - 200 °C and the preparation can take place in static or in dynamic mode. Finally, in step a) the template is optionally removed by a thermal treatment procedure known as step calcination procedure. The temperature of the treatment is in the range of 350 - 900 °C removed. The template may alternatively be removed in step i) if it was not removed in step a) but preferably the template is removed in step a).

In step b) the mesoporous molecular sieve gel is prepared from silicon sources, optional aluminium sources, and surfactant (S). The silicon sources are selected from silicon compounds having an organic group and from inorganic silicon sources. Those silicon sources having an organic group are tetraethoxy silane (TEOS), tetramethylammonium silicate, tetraethylammonium silicate etc. The inorganic silicon sources are sodium silicate, water glass, colloidial silica, solid silica and fumed silica. The aluminium source is selected from aluminium sulphate (Al₂(SO₄)₃.18H₂O), hydrated aluminium hydroxides, aluminates, aluminium isoproxide and alumina. The surfactant is selected in order to obtain the desired mesoporous phases. Suitable surfactants used are alkyltrimethyl ammonium halide compounds with the general formula CₙH₂ₙ₊₁ (CH₃)₃∗NX, where n = 12 to 18, X= Cl, Br. Preferably the surfactant is selected from the group consisting of n-hexadecyltrimethyl ammonium bromide, n-hexadecyltrimethyl ammonium chloride, cetyltrimethylammonium bromide and cetyltriethylammonium bromide. The temperature in step b) ranges between 20 and 100 °C and the preparation takes place under stirring.

In step c) the zeolite nuclei prepared in step a) are introduced to the molecular sieve gel under stirring. The formed mixture is homogenised and the zeolite nuclei are dispersed. For adjusting the acidity of the product, additional aluminium source is added. This additional aluminium source is an aluminium source having an organic ligand selected from aluminium alkoxides, preferably aluminium isopropoxide. The stirring rate in step c) ranges between 50 and 1000 rpm. The treatment time is between 10 - 500 minutes.

In step d) the gel is ripened under stirring. The stirring rate is 200 - 1000 rpm and the time of the gel ripening is between 30 - 1800 minutes.

In step e) the hydrothermal synthesis is performed at a temperature between 100-200 °C. The time for the hydrothermal synthesis can vary between 10 h - 300 h depending the material desired. The hydrothermal synthesis is performed in dynamic mode under continuous stirring of the mixture until crystals are formed.

In step f) the crystals from step e) are recovered, for example by filtration or by another method known to be state of the art. If necessary, the pH of the mixture is adjusted to 6 - 8 before the recovery, such as filtration.

In step g) the solid product obtained in step f) is thoroughly washed using for example water as the washing liquid. The temperature of the water is from room temperature to 60 °C. The washing is finished when all undesired, soluble materials are removed from the solid product.

In step h) the solid product is dried for removal of solvent by the methods known to be state of art.

In step i) the surfactant (S) is partly or totally removed by a thermal treatment procedure known as step calcination procedure. The templetate (R) may optionally be removed in step i) simultaneously with the removal of the surfactant. The temperature of the treatment is in the range of 350 - 900 °C. The heating rate ranges between 0.2 and 10 °C/min. The atmosphere in the treatment is oxidising and in the final step the material is typically treated in air. A mesoporous molecular sieve embedded with a zeolite catalyst is obtained.

In the manufacturing method a gel solution is prepared of the mesoporous molecular sieve, then the zeolite nucleating agent is added at suitable synthesis conditions and an aluminium source is substituted with zeolite nucleating agent. Suitably the aluminium source is an aluminium alkoxide and preferably aluminium isopropoxide.

Preferably the surfactant is n-hexadecyltrimethylammonium bromide, n-hexadecyltrimethyl ammonium chloride, cetyltrimethylammonium bromide or cetyltriethylammonium bromide.

Preferably distilled water or deionized water is used as a solvent and in washing of the material.

Zeolite nuclei are aluminosilicate precursors free of structure directing agents, and they may be partially or fully crystalline. Because of the variation in crystal size they may be or may not be detected by XRD. However, their morphology can be observed by scanning electron microscopy. The zeolite nuclei have a meta-stable phase, which in the presence of a surfactant, during the synthesis of the catalytic material obtained by the method according to the invention accomplishes chemical bonding with the walls of the mesoporous molecular sieve.

After the intensive dispersion of the aluminosilicate nuclei to the mesoporous molecular sieve gel solution, in the presence of a surfactant and during the gel ripening period, a nuclei-surfactant mesophase complex is formed, which strengthens and enhances the chemical bonding and crystallinity of the walls of the mesoporous material.

The aluminosilicate precursor for the zeolite nuclei can be prepared for zeolite structure types such as MFI, BEA, TON, MOR, MWW, AEF and FAU from known state of the art (EP 23089, US Patent 3308069, EP 102716, EP 23089). Two examples of the preparation of aluminosilicate precursors for zeolite nuclei of structures MFI and BEA are given here. It is however obvious that the other mentioned zeolites are equally suitable.

The zeolite nuclei, prepared from an aluminosilicate precursor, are suitably used in the gel preparation. During the gel ripening period chemical interactions and bonding via process of nucleation take place. Gel ripening accelerates the nucleation process and secondary nucleation may also occur on the zeolite nuclei forming thereby a complex "zeolite nuclei-surfactant mesophase", which enhances the chemical nature of bonding between the micro and mesophases. Microphases are responsible for the formation of the zeolite structure, and mesophases for the formation of the mesoporous structure.

Formation of "zeolite nuclei-surfactant mesophase" is favoured when the zeolite nuclei is introduced after the addition of the surfactant in alkaline media or soaking the zeolite nuclei in an aqueous solution of the surfactant prior to its addition, followed by gel ripening period.

The order of introducing the reagents, specially the surfactant and zeolite nuclei, pretreatment of the zeolite nuclei and gel ripening process are important for the creation of the chemical nature of bonding between the microporous and mesoporous molecular sieve material. In order to obtain the high acidity mesoporous molecular sieve embedded with a zeolite materials, an aluminium source is introduced after the addition of zeolite nuclei but prior to gel ripening period.

Vigorous stirring after introducing of the zeolite nuclei during the gel preparation is significant in order to increase the homogenity and dispersion of zeolite nuclei in the gel solution.

The obtained catalytic materials may optionally be transformed to the corresponding proton forms via ammonium ion-exchange and calcination. A suitable source material for ammonium ion-exchange is an ammonium salt like ammonium nitrate or ammmonium chloride. The catalytic materials are treated in an aqueous solution of the ammonium salt at temperatures between 25 - 80 °C for a suitable time interval like 1 to 6 hours. The ammonium cations replace the alkali or alkaline cations of the materials during the treatment. The degree of the ion-exchange can be varied by changing the time of the treatment, concentration of ammonium solution and temperature. After the ion-exchange treatment, the obtained material is dried and calcined for decomposing the ammonium ions to proton and ammonia.

Modifications of catalytic material, the mesoporous molecular sieve embedded with a zeolite, can be carrier out by methods selected from a group consisting of precipitation, deposition, encapsulation, and selective removal. Both impregnation and ion exchange are deposition methods. In impregnation, the deposition is carried out from liquid phase and adsorption, ion exchange and selective reaction may take place on or with the surface of the support. During the removal of the liquid, crystallites rather than monolayers are formed on the surfaces. In ion exchange diluted solutions are used, and the desired metal cation is exchanged from the solution to the material replacing the cation or proton of the solid material. The procedures and choice of method for modification depend on the target reactions. Generally, ion exchange method is preferred when lower loading and higher dispersion of metal is needed.

The removal of surfactant after the completion of the synthesis is necessary to obtain the mesoprous molecular sieves embedded with a zeolite with high surface area and acidity. The calcination temperature, heating rate and duration may influence the surface area, pore size distribution and location of aluminium in the framework. Very large surface area, determined by nitrogen adsorption and varying strong acidity, determined by TPD of ammonia, of the synthesized mesoporous molecular sieve embedded with a zeolite confirms that the step calcination procedure is a very suitable method for surfactant removal.

The removal of template from the mesoporous molecular sieves embedded with a zeolite is also performed by step calcination procedure.

The synthesis of the mesoporous material may be carried out with or without additional aluminium sources. Only one template is needed in the synthesis of the catalytic material.

The catalytic material may be incorporated in or on a carrier using any methods known in the art.

The method of synthesis results in increased crystallinity of the pore walls by chemically bonding the zeolite material in the mesoporous material and thereby introducing the desired zeolite properties and simultaneously maintaining the mesoporous structure intact. In this method only one type of template in the gel solution for the synthesis of the product is needed.

Small crystals of a zeolite are used as "nuclei" in the synthesis of the mesoporous material and it is possible to vary the concentration of the "nuclei" and the size of the zeolite crystals. This results in the increase of the concentration of zeolite nuclei and in embedding of large amount of the microporous structure in the mesoporous molecular sieve and increased crystallinity of the mesoporous wall. It also influences the thermal and hydrothermal stability and acidic properties of the material. Variation in the size of the zeolite nuclei may influence the shape selective property of the material.

The X-ray powder diffraction patterns, scanning electron microscopy and nitrogen adsorption characterization results confirm the high thermal and hydrothermal stability of the mesoporous molecular sieve embedded with a zeolite obtained by the method according to the invention, such as MFI, BEA, MWW and MOR structures.

Further, the complete or almost complete regeneration of the used catalysts from different hydrocarbon conversion reactions, like n-butane and 1-butene isomerization and 1-decene oligomerization, and retaining of the catalytic activities also indicates the stability of catalytic material.

The manufacturing method makes it possible to design intrinsic acidic properties in the mesoporous molecular sieve. The intrinsic acidic properties of the mesoporous molecular sieve materials can be designed using a source of aluminium and varying the Si/Al ratio of the gel solution and different zeolite nuclei. Characterization results of H-MM5, H-MMBE and H-MMMW catalysts by TPD of ammonia and different test reactions like n-butane isomerization confirm the success in designing these materials exhibiting varying acidities.

The pore walls of the mesoporous material are amorphous in MCM-41, but with the introduction of zeolite they exhibit increased crystallinity. The zeolite unit cell in the product obtained by the method according to the invention is different from the one in a mechanical mixture of a zeolite and a mesoporous molecular sieve, and the mesoporous molecular sieve unit cell is larger than that in a mechanical mixture.

A further essential feature of the product is that the majority of the zeolite phase is chemically bonded to the mesoporous molecular sieve. The product is thermally stable at a temperature of at least 900°C in the presence of air.

The mesoporous molecular sieve embedded with a zeolite obtained by the method according to the invention and the method for the manufacture of such material are significantly different from the ones disclosed in the state of the art and exhibit several advantages.

The new MM5 mesoporous molecular sieve embedded with MFI structure is thermally stable up to at least 1000 °C and MMBE to at least 900 °C, as can be seen from XRD and SEM figures and surface area measurements.

The transformation of the cationic forms of MM5, MMBE, MMMW and MMMO, such as the Na-forms presented in the examples, to the corresponding proton forms of MM5, MMBE, MMMW and MMMO by ion-exchange with aqueous solution of ammonium nitrate, followed by drying at 100 °C and calcination at 500 °C did not change the structure as was shown by XRD patterns, indicating the hydrothermal stability of the material. It is known that the structure of MCM-41 collapses after bringing it in contact with water at high temperatures.

Metal modifications of MM5, MMBE, MMMW and MMMO, such as noble metal modifications and particularly platinum modifications of the examples, manufactured using aqueous solutions of hexachloroplatinic acid at 80 °C for 24 hours, followed by drying at 100 °C and calcination at 450 °C did not influence the structures of MM5 and MMBE as shown by XRD patterns. This indicates that the material is stable in an acidic medium.

The proton form of the mesoporous molecular sieve embedded with MFI structure showed very high activity in n-butane and 1-butene isomerization reactions. The H-MM5 catalysts showed an increase in n-butane conversion with an increase in the acidity.

The proton forms of the mesoporous molecular sieve embedded with MFI and BEA structures showed very high activity in the dimerization of 1-olefins. The H-MM5 and H-MMBE catalysts showed an increase in 1-decene conversion, with an increase in the acidity. The proton form of the mesoporous molecular sieve embedded with MFI structure showed very high activity in the dimerization of isobutene and the catalyst was not deactivated.

The H-MM5 and H-MMBE catalytic materials were fully regenerated in presence of air. The regenerated materials showed almost the same catalytic activity in n-butane and 1-butene isomerization and 1-decene oligomerization as the fresh catalyst. It is well known that one of the major problems of MCM-41 catalyst is the regeneration i.e. the mesoporous structure collapses after regeneration. The retaining of the catalytic activity in the new mesoporous molecular sieve embedded with a zeolite materials in both the reactions unequivocally shows that the structure after the regeneration is stable.

Pt-MM5 showed very high conversion in n-butane isomerization and the catalytic material after regeneration retained its catalytic activity.

Pt-MMBE showed a high selectivity to ring opening products.

Thus post-synthesis modification is not needed for the MM5 and MMBE mesoporous materials to increase the thermal and hydrothermal stability. The high thermal and hydrothermal stability of the materials produced according to the invention, are attributed to embedding of the zeolite, such as MFI and BEA structure to the walls of mesoporous molecular sieves by using method as described above.

This new group of mesoporous materials can be applied as catalysts in dimerization of olefins, oligomerization of olefins, isomerization of olefins, cracking of hydrocarbons, alkylation of aromatics, aromatization of light hydrocarbons, etherification, dehydration and ring opening reactions without further modifications of the active material. The metal modified material showed a high activity in the isomerization of light paraffins. Similarly, the metal modified materials may also be active in the isomerization of long chain paraffins, hydrogenation, hydrocracking, hydrodesulfurization, hydrodeoxygenation, hydrodenitrogenation, dehydrogenation, reforming, Fisher-Tropsch and oxidation reactions when modified using the manners known to be state of art. The metal in the catalyst can be in metallic, oxide or in sulfide form or in any other form when modified with the manner known to be state of art.

The material obtained by the method according to the invention can also be utilised in various separation techniques like in adsorption, absorption or in selective removal.

The following illustrative examples provide better understanding of the invention and its practical embodiments, however it is evident to a man skilled in the art that the scope of the invention is not limited to these examples in any way.

### EXAMPLES

### Example 1 (Comparative)

### Manufacture of ZSM-5 zeolite according to US 3,926,784

The starting materials were aluminium silicate, aluminium sulphate, triisopropylamine bromide (TPABr), sodium chloride, sulphuric acid and water.

Solution A was prepared by mixing of 3.5 g of aluminium silicate with 4.4 1 water. Solution B was prepared by mixing 107 g of aluminium sulphate, 438g of TPABr, 1310g of NaCl, 292 g of H2SO4 and 6 1 water. The solutions were introduced to a reactor under stirring with the stirring speed of 250 r/min. The temperature was gradually increased to 100 °C and the pressure was increased to 8 bar. The reaction was under stirring for 6 days. The reactor was cooled. The formed solid product (ZSM-5) was filtered, washed with warm water and dried at 110 °C overnight. The product was calcined for the removal of the template, ion exchanged with ammonium nitrate and calcined for preparing the proton form of the zeolite (H-ZSM-5).

### Examples 2-4 (Comparative)

### Manufacture of MSA type materials according to EP 0 784 652

The starting materials used in the synthesis of MSA type materials were aluminium isopropoxide (Al-i-C₃H₇O)₃, tetraethyl orthosilicate (Si(C₂H₅O)₄) and aqueous solution of tetrapropyl ammonium hydroxide (TPA-OH).

TPA-OH, (Al-i-C₃H₇O)₃ and water were mixed at 60 °C for 40 minutes. The obtained solution was heated to 85 °C and a clear solution was formed. Then liquid Si(C₂H₅O)₄ was added via a drop funnel. The obtained mixture was stirred for 3 hours. The reaction mixture was cooled under continuous stirring for 20 hours. After cooling the formed alcohol and water was evaporated and the solid gel was dried at 100 °C. The dry solid was milled and calcined at 550 °C for 8 hours.

In the following table 3 the preparation and properties of the obtained MSA type catalysts are provided.

**Table 3.**

| **Synthesis parameters:** | **Example 2 MSA-1** | **Example 3 MSA-2** | **Example 4 MSA-3** |
|---|---|---|---|
| Si/Al (mol/mol) | 50 | 12 | 5 |
| TPA-OH/Water (mol/mol) | 0.18 | 0.18 | 0.18 |

| **Product properties:** | | | |
|---|---|---|---|
| Si/Al (mol/mol) | 54 | 11.5 | 4.8 |
| BET surface area (m²/g) | 650 | 440 | 310 |
| Surface area of micropores (m²/g) | 380 | 340 | 210 |
| Average pore size (nm) | 1.4 | 1.4 | 1.4 |
| Surface area of mesopores (m²/g) | 270 | 100 | 80 |

### Example 5 (Comparative)

### Manufacture of mesoporous molecular sieve H-MCM-41

The synthesis of Na-MCM-41 was carried out by preparing solutions A, B and C. Solution A was prepared by mixing fumed silica with distilled water with continuous stirring for 15 minutes. Solution B was prepared by adding tetramethylammonium silicate to sodium silicate with continuous stirring and the mixture was stirred for 20 minutes. Solution C was prepared by dissolving tetradecyltrimethyl ammonium bromide in distilled water with stirring for 20 minutes. Solution B was added to Solution A slowly (in 15 min) with stirring and after the addition Solution B the mixture was stirred for further 20 minutes. Solution C was slowly (20 min) added to the mixture of A and B with stirring and after the addition of solution C the mixture was further stirred for 20 minutes. Then aluminium isopropoxide was added to the gel solution (A +B +C) under stirring and the obtained mixture was gel ripened for two hours with stirring. The pH was controlled and the gel was introduced in a teflon cup, which was inserted in an autoclave. The synthesis was carried out for 48 h at 100 °C. After completion of the synthesis, the reactor was quenched and the mesoporous material was filtered and washed with distilled water. The obtained Na-MCM-41 was dried at 110 °C and calcined at 550 °C for 10 h. The sodium form of Na-MCM-41 was ion-exchanged with aqueous 1 M ammonium nitrate solution for 2 h at 80 °C and then the obtained NH₄-MCM-41 was washed with distilled water, dried and calcined.

### Examples 6-8

### Mesoporous molecular sieve embedded with MFI zeolite structure Preparation of the MFI zeolite nuclei

Three different solutions A, B and C were made for MFI zeolite nuclei preparation. Solution A was prepared by adding 10.5 g of fumed silica to 81.2 ml of distilled water. Solution B was prepared by dissolving 2.2 g of NaOH and 0.3 g of Al (OH)₃ in 9.4 ml of distilled water. Solution B was added to Solution A and the obtained gel mixture stirred for 20 minutes. Solution C was prepared by dissolving 3.7 g of tetrapropyl ammonium bromide in 3.8 ml of water and stirring for 20 minutes. Solution C was added to the gel mixture (A+B) and stirred for 15 minutes and 55 ml of water was added. The obtained gel mixture was further stirred for 20 minutes. Synthesis was carried out at 150 °C for 18 h. After the completion of synthesis the product was filtered, washed with distilled water, dried and calcined and MFI zeolite nuclei were obtained.

### Example 6a

### Synthesis of mesoporous molecular sieve embedded with MFI structure, Na-MM5-96h-4ZS, without aluminium source

The synthesis of Na-MM5-96h-4ZS was carried out by preparing solutions A, B and C. Solution A was prepared by mixing 8.3 g of fumed silica with 51.7 g of distilled water with continuous stirring (r.m.p. 196) for 20 minutes. Solution B was prepared by adding 18.1 g of tetramethyl ammonium silicate to 11.7 g of sodium silicate with continuous stirring (r.m.p 180) and the mixture was stirred for 20 minutes. Solution C was prepared by dissolving 26.3 g of tetradecyltrimethyl ammonium bromide in 174.3 g of distilled water with vigorous stirring (r.m.p. 336) for 20 minutes. Solution B was added to Solution A slowly (in 15 min) with vigorous stirring (r. m. p. 320) and after the addition of solution B the mixture was stirred for further 20 min. Solution C was added to the mixture (A + B) slowly (20 min) with vigorous stirring (r. m. p. 336) and after the addition of solution C the mixture was further stirred for 20 minutes.

Then 4.2 g of MFI nuclei manufactured above were dispersed to the gel solution (A+B+C) under vigorous stirring (r.m.p. 340) for 20 minutes. The homogenisation of the dispersed MFI was carried out by further vigorous stirring (340 r.m.p.) of the gel for 35 minutes. Then the gel solution was ripened for three hours at ambient temperature with stirring (r.m.p 180). pH of the gel was controlled and the gel was introduced in a teflon cup which was then inserted in an autoclave. The synthesis was carried out for 96 h at 100 °C. After completion of the synthesis, the reactor was cooled for 30 min and the obtained mesoporous molecular sieve material imbedded with MFI structure was mixed with distilled water, filtered and washed thoroughly with distilled with water for 3 h. The Na-MM5-96h-4ZS was dried and calcined at 450 °C using step calcination procedure for 10 h in a muffle oven.

### Example 6b

### Preparation of H-MM5-96h-4ZS, proton form of the above material

10 g of Na-MM5-96h-4ZS (sodium form, prepared above) was ion-exchanged with an aqueous solution of 1 M ammonium nitrate or ammonium chloride for 24 h at ambient temperature. After ion-exchange the obtained NH₄-MM5-96h-4ZS was washed thoroughly with distilled water, dried and calcined using step calcination procedure in a muffle oven at 450 °C.

The XRD pattern of the obtained H-MM5-96h-4ZS was similar to that of Na-MM-5-96h-4ZS indicating that the aqueous treatment of the mesoporous material obtained by the present method and subsequent thermal treatment did not influence the stability of the structure.

### Example 7

### Synthesis of mesoporous molecular sieve embedded with MFI structure, Na-MM5-96h-4ZS-2AI, using aluminium source

### Example 7a

### Synthesis of Na-MM5-96h-4ZS-2AI

The synthesis of Na-MM5-96h-4ZS-2AI was carried out by preparing solutions A, B and C. Solution A was prepared by mixing 8.3 g of fumed silica with 51.7 g of distilled water with continuous stirring (r.m.p. 196) for 20 minutes. Solution B was prepared by adding 18.1 g of tetramethylammonium silicate to 11.7 g of sodium silicate with continuous stirring (r.m.p 180) and the obtained mixture was stirred for 20 minutes. Solution C was prepared by dissolving 26.3 g of tetradecyl trimethyl ammonium bromide in 174.3 g of distilled water with vigorous stirring (r.m.p. 336) for 20 minutes. Solution B was added to Solution A slowly (in 15 min) with vigorous stirring (r. m. p. 320) and after the addition of solution B the mixture was stirred for further 20 min. Solution C was added to the mixture (A + B) slowly (20 min) with vigorious stirring (r. m. p. 336) and after the addition of solution C it was further stirred for 20 minutes.

Then 4.2 g of MFI nuclei was dispersed to the gel solutions (A +B +C) under vigorous stirring (r.m.p. 340) for 20 minutes. The homogenisation of the dispersed MFI was carried out by further vigorous stirring (r. m. p. 340) of the gel for 35 minutes. Then 2.3 g of aluminium isopropoxide was added and stirred for 20 min. The obtained gel was allowed to ripen for three hours with stirring (r.m.p 180). pH of the gel was controlled and the gel was introduced in a teflon cup which was then inserted in an autoclave. The synthesis was carried out for 96 h at 100 °C. After completion of the synthesis, the reactor was quenched and the obtained mesoporous material was filtered and washed thoroughly with distilled with water. The obtained Na-MM5-96h-4ZS-2AI was dried and calcined using step calcination procedure in a muffle oven.

### Example 7b

### Synthesis of H-MM5-96h-4ZS-2AI

10 g of Na-MM5-96h-4ZS-2AI (prepared above) was ion-exchanged with 1 M ammonium nitrate aqueous solution for 24 h at room temperature. After the ion-exchange the mesoporous material was washed thoroughly, dried at and calcined for four hours using step calcinations procedure in a muffle oven at 450 °C. The XRD pattern of the obtained H-MM5-96h-4ZS-2AI was similar to that of Na-MM-5-96h-4ZS-2AI indicating that the aqueous treatment of the mesoporous material obtained by the present method and subsequent thermal treatment did not influence the stability of the structure.

### Example 8

### Synthesis of mesoporous molecular sieve embedded with MFI structure, Na-MM5-96h-4ZS-2AI-35, using aluminium source

### Example 8a

### Synthesis of Na-MM5-96h-4ZS-2AI-35

The synthesis of Na-MM5-96h-4ZS-2AI-35 was carried out by preparing solutions A, B and C. Solution A was prepared by mixing 4.5 g of fumed silica with 51.7 g of distilled water with continuous stirring (r.m.p. 196) for 20 minutes. Solution B was prepared by adding 18.1 g of tetramethylammonium silicate to 11.7 g sodium silicate with continuous stirring (r.m.p 180) and the obtained mixture was stirred for 20 minutes. Solution C was prepared by dissolving 26.3 g of tetradecyl trimethyl ammonium bromide in 174.3 g of distilled water with vigorous stirring (r.m.p. 336) for 20 minutes. Solution B was added to Solution A slowly (in 15 min) with vigorous stirring (r. m. p. 320) and after the addition of Solution B the obtained mixture was stirred for further 20 min. Solution C was added to the mixture (A + B) slowly (20 min) with vigorous stirring (r. m. p. 336) and after the addition of solution C the mixture was further stirred for 20 minutes. Then 4.2 g of MFI nuclei, prepared in example 6, were dispersed to the gel mixture (A +B +C) under vigorous stirring (r.m.p. 340) for 20 minutes. The homogenisation of the dispersed MFI was carried out by further vigorous stirring (r. m. p. 340) of the gel for 35 minutes. Then 2.3 g of aluminium isopropoxide was added to the mixture and stirred for 20 min. Then the gel was allowed to ripen for three hours with stirring (r.m.p 180). pH of the gel was controlled and the gel was introduced in a teflon cup which was then inserted in an autoclave. The synthesis was carried out for 96 h at 100 °C. After completion of the synthesis, the reactor was quenched and the obtained mesoporous material was filtered and washed thoroughly with distilled with water. The obtained Na-MM5-96h-4ZS-2AI-35 was dried and calcined using step calcination procedure in a muffle oven at 450 °C.

### Example 8b

### Preparation of H-MM5-96 h-4ZS-2AI-35

10 g of Na-MM5-96h-4ZS-2AI-35 (manufactured above) was ion-exchanged with 1 M ammonium nitrate aqueous solution for 24 h at room temperature. After the ion-exchange the mesoporous material was washed thoroughly, dried and calcined using step calcination procedure in a muffle oven at 450 °C.

The XRD pattern of the H-MM5-96 h-4ZS-2AI-35 was similar to that of Na-MM-5-96 h-4ZS-2AI-35 indicating that the aqueous treatment of the present mesoporous material obtained by the present method and subsequent thermal treatment did not influence the stability of the structure.

### Example 9

### Platinum modified MM5 material, Pt-H-MM5-96 h-4ZS-2AI

5 g of H-MM5-96h-4ZS-2AI was loaded with 2 wt % of Pt using impregnation method. 2 wt % Pt impregnation was performed in a rotary evaporator at 80 °C for 24 h using aqueous solution of hexachloroplatinic acid. The 2 wt % Pt impregnated MM-5-96h-2AI was dried at 100 °C and calcined at 450 °C. The XRD pattern Pt-H-MM-5-96h-4ZS-2AI presented in Figure 5 was similar to those of parent Na-MM-5-96h-2AI indicating the hydrothermal stability of the mesoporous molecular sieve embedded with a zeolite obtained by the present method.

### Example 10

### Preparation of platinum modified MM5 material, Pt-H-MM5-96h-4ZS-2AI-35

5 g H-MM5-96h-4ZS-2AI-35 was loaded with 2 wt % Pt using impregnation method. 2 wt % Pt impregnation was performed in a rotary evaporator at 80 °C for 24 h using aqueous solution of hexachloroplatinic acid. The 2 wt % Pt impregnated MM-5-96h-2 AI-35 was dried at 100 °C and calcined at 450 °C.

The XRD pattern Pt-H-MM-5-96h-4ZS-2AI-35 was similar to that of parent Na-MM-5-96h-2AI-35 indicating the hydrothermal stability of the present mesoporous molecular sieve embedded with a zeolite obtained by the present method.

### Examples 11-13

### Preparation of mesoporous materials embedded with BEA-structure

### Preparation of BEA zeolite nuclei

7.8 g of NaAlO₂ was mixed with 60 ml of distilled water under stirring for 10 minutes and to this solution 74 g of tetraethyl ammonium hydroxide (TEA-OH, 40 %) was added and stirred for 20 minutes. 145.4 g of colloidal silica was added to the above solution and stirred for 25 minutes.

The obtained gel was put in the autoclave and inserted in the teflon cups. The synthesis was carried out at 150 °C for 65 h in static mode. After the completion of synthesis the product was filtered, washed with distilled water, dried at 110 °C and calcined at 550 °C for 7 hours and the BEA zeolite was obtained.

### Example 11a

### Synthesis of mesoporous molecular sieve embedded with BEA structure, Na-MMBE-96h-4B, without aluminium source

The synthesis of Na-MMBE-96h-4B was carried out by preparing of solutions A, B and C. Solution A was prepared by mixing 8.3 g of fumed silica with 51.7 g of distilled water with continuous stirring (r.m.p. 196) for 20 minutes. Solution B was prepared by adding 18.1 g of tetramethylammonium silicate to 11.7 g of sodium silicate with continuous stirring (r.m.p 180) and the mixture was stirred for 20 minutes. Solution C was prepared by dissolving 26.3 g of tetradecyltrimethyl ammonium bromide in 174.3 g of distilled water with vigorous stirring (r.m.p. 336) for 20 minutes. Solution B was added to Solution A slowly (in 15 min) with vigorous stirring (r. m. p. 320) and after the addition of solution B the obtained mixture was stirred for further 20 min. Solution C was added to the mixture A + B slowly (20 min) with vigorous stirring (r. m. p. 336) and after the addition of solution C the mixture was further stirred for 20 minutes.

Then 3.7 g of BEA zeolite nuclei precursor, prepared above was added to the gel mixture (A +B +C) under vigorous stirring (r.m.p. 350) and the gel was ripened for three hours with stirring (r.m.p 180). pH of the gel was controlled and the gel was introduced in a teflon cup which was then inserted in an autoclave.

The synthesis was carried out for 96 h at 100 °C. After completion of the synthesis, the reactor was quenched and the obtained mesoporous material was filtered and washed thoroughly with distilled with water. The obtained Na-MM-BE-96h-4B was dried and calcined using step calcination procedure.

### Example 11b

### Preparation of H-MMBE-96h-4B

10 g of Na-MMBE-96h-4B (manufactured above) was ion-exchanged with 1 M ammonium nitrate solution for 24 h at room temperature. After the ion-exchange the mesoporous material was washed thoroughly, dried and calcined using step calcination procedure.

The XRD pattern of H-MMBE-96h-4B was similar to that of Na-MMBE-96h-4B indicating that the aqueous treatment of the present mesoporous material obtained by the present method and subsequent thermal treatment did not influence the stability of the structure.

### Example 12

### Synthesis of mesoporous molecular sieve embedded with BEA structure, Na-MMBE-96h-4B-2AI, with aluminium source.

### Example 12a

### Synthesis of Na-MMBE-96h-4B-2AI

The synthesis of Na-MM-BE-96 h-4B-2AI was carried out by preparing of solutions A, B and C. Solution A was prepared by mixing 8.3 g of fumed silica with 51.7 g of distilled water with continuous stirring (r.m.p. 196) for 20 minutes. Solution B was prepared by adding 18.1 g of tetramethyl ammonium silicate to 11.7 g sodium silicate with continuous stirring (r.m.p 180) and the obtained mixture was stirred for 20 minutes. Solution C was prepared by dissolving 26.3 g of tetradecyl trimethyl ammonium bromide in 174.3 g distilled water with vigorous stirring (r.m.p. 336) for 20 minutes. Solution B was added to Solution A slowly (in 15 min) with vigorous stirring (r. m. p. 320) and after the addition of solution B the mixture was stirred for further 20 min. Solution C was added to the mixture (A + B) slowly (20 min) with vigorous stirring (r. m. p. 336) and after the addition of solution C the mixture was further stirred for 20 minutes.

Then 3.7 g of BEA zeolite nuclei precursor (prepared above) was added to the gel mixture (A +B +C) under vigorous stirring (r.m.p. 350) and stirred for 25 min and then 1.9 g of aluminium isopropoxide was added and stirred for 20 min. The obtained gel was allowed to ripen for three hours with stirring (r.m.p 180). pH of the gel was controlled and the gel was introduced in a teflon cup which was then inserted in an autoclave. The synthesis was carried out for 96 h at 100 °C. After completion of the synthesis, the reactor was quenched and the mesoporous material was filtered and washed thoroughly with distilled with water. The obtained Na-MMBE-96h-4B-2AI was dried and calcined using step calcination procedure for 10 h.

### Example 12b

### Preparation of H-MMBE-96 h-4B-2AI

10 g of Na-MMBE-96h-4B-2AI (manufactured above) was ion-exchanged with 1 M ammonium nitrate aqueous solution for 24 h at ambient temperature. After the ion-exchange the mesoporous molecular sieve material was washed thoroughly with distilled water, dried and calcined in a muffle oven using step calcination procedure.

The XRD pattern of the H-MMBE-96h-4B-2AI was similar to that of Na-MMBE-96 h-4B-2AI indicating that the aqueous treatment of the mesoporous material obtained by the present method and subsequent thermal treatment did not influence the stability of the structure.

### Example 13

### Synthesis of mesoporous molecular sieve embedded with a BEA structure, Na-MMBE-96h-4B-2AI35, with aluminium source

### Example 13a

### Synthesis of Na-MMBE-96 h-4B-2AI-35

The synthesis of Na-MMBE-96h-4B-2AI-35 was carried out by preparing of solutions A, B and C. Solution A was prepared by mixing 4.4 g of fumed silica with 51.7 g of distilled water with continuous stirring (r.m.p. 196) for 20 minutes. Solution B was prepared by adding 18.1 g of tetramethyl ammonium silicate to 11.7 g of sodium silicate with continuous stirring (r.m.p 180) and the mixture was stirred for 20 minutes. Solution C was prepared by dissolving 26.3 g of tetradecyl trimethyl ammonium bromide in 174.3 g of distilled water with vigorous stirring (r.m.p. 336) for 20 minutes. Solution B was added to Solution A slowly (in 15 min) with vigorous stirring (r. m. p. 320) and after the addition of solution B the mixture was stirred for further 20 min. Solution C was added to the mixture (A + B) slowly (20 min) with vigorous stirring (r. m. p. 336) and after the addition of solution C the mixture was further stirred for 20 minutes. Then 3.7 g of BEA zeolite nuclei precursor (prepared above) was added to the gel mixture (A +B +C) under vigorous stirring (r.m.p. 350) and the mixture was stirred for 25 min. Then 1.9 g of aluminium isopropoxide was added and stirred for 20 min. The obtained gel was allowed to ripen for three hours with stirring (r.m.p 180). pH of the gel was controlled and the gel was introduced in a teflon cup which was then inserted in an autoclave. The synthesis was carried out for 96 h at 100 °C. After completion of the synthesis, the reactor was quenched and the mesoporous material was filtered and washed thoroughly with distilled with water. The obtained Na-MMBE-96h-4B-2AI-35 was dried and calcined using step calcination procedure.

### Example 13b

### Preparation of H-MMBE-96h-4B-2AI-35

10 g of Na-MMBE-96h-4B-2AI-35 (prepared above) was ion-exchanged with 1 M ammonium nitrate aqueous solution for 24 h at ambient temperature. After the ion-exchange the mesoporous molecular sieve material was washed thoroughly with distilled water, dried and calcined in a muffle oven using step calcination procedure.

The XRD pattern of H-MMBE-96h-4B-2AI-35 was similar to that of Na-MMBE-96 h-4B-2AI-35 indicating that the aqueous treatment of the mesoporous material obtained by the present method and subsequent thermal treatment did not influence the stability of the structure.

### Example 14

### Platinum modified MMBE material, Pt-H-MMBE-96h-4B-2AI

5 g of H-MMBE-96h-4B-2AI-35 (prepared above) was loaded with 2 wt % Pt using impregnation method. 2 wt % Pt impregnation was carried out in a rotator evaporator at 80 °C for 24 h using aqueous solution of hexachloroplatinic acid. The 2 wt % Pt impregnated H-MMBE-96h-2AI was dried and calcined. The XRD pattern of Pt-H-MMBE-96h-4B-2AI was similar to the one of the parent Na-MM-BE-96h-4B-2AI indicating the hydrothermal stability of the mesoporous molecular sieve obtained by the present method. Further more Pt modification of H-MMBE-96h-4B-2AI did not influence the parent structure.

### Example 15

### Preparation of platinum modified MMBE material Pt-H-MMBE-96h-4B-2AI-35

5 g of H-MMBE-96h-4B-2AI-35 was loaded with 2 wt % Pt using impregnation method. 2 wt % Pt impregnation was carried out in a rotator evaporator at 80 °C for 24 h using aqueous solution of hexachloroplatinic acid. The 2 wt % Pt impregnated H-MMBE-96h-2AI-35 was dried and calcined. The XRD pattern of Pt-H-MMBE-96h-4B-2AI-35 was similar to that of the parent Na-MMBE-96h-4B-2AI-35 indicating the hydrothermal stability of the mesoporous molecular sieve obtained by the present method. Further more Pt modification of H-MMBE-96h-4B-2AI-35 did not influence the parent structure.

### Examples 16-18

### Preparation of Pt-H-MMBE with ion exchange

2 g of each H-MMBE materials: H-MMBE-96h-4B (example 16), H-MMBE-96h-4B-2AI (example 17) and H-MMBE-96h-4B-2AI-35 (example 18), was weighed to 2 1 flasks. 1 l of ion-exchanged water was added. Reflux condenser was placed on top of the flask. The flask was placed in a water bath, temperature 70°C and shake 110. Flask was kept in these conditions for one hour. Then the reflux condenser was replaced with dropping funnel with an air exhaust port. 52 ml of 0.01 M Pt-solution was measured to the dropping funnel. The Pt-solution was dropped slowly (about 15 drops per minute) to the flask, temperature 70°C, shake 110. The Pt-adding took 53 minutes. The dropping funnel was replaced with a reflux condenser and the flask was left to these conditions for 24 hours.

The reaction mixture from the flask was filtered with suction using sintered glass crucible. The obtained material was washed in the flask with one 1 of ion-exchanged water and filtered again. This was done twice. After the second wash the sintered glass crucible with the material was placed to an oven at a temperature of 80°C for 16 hours.

After 16 hours drying material was transferred to a crucible and calcined in an oven. Temperature was elevated from 21°C to 300°C with rate 0.2°C/min.

### Examples 19 - 28

### Mesoporous molecular sieve embedded with MWW zeolite structure

### Preparation of MWW zeolite nuclei

Two solutions A and B were made for MWW zeolite nuclei preparation. Solution A was prepared by adding 87.58 g of sodium silicate to 42 ml of distilled water under stirring for 15 minutes and to this solution was added 16.7 g of hexamethylene dropwise over a period of 25 minutes and solution stirred for 20 minutes. Solution B was prepared by adding 7.35 g of concentrated sulphuric acid to 224 ml of distilled water and stirring for 10 minutes after which 8.9 g of aluminium sulphate was added and stirred for 20 minutes. Solution B was added to Solution A slowly and with vigorous stirring. The gel was introduced in Teflon cups and inserted in 300 ml autoclaves. The synthesis was carried out at 150 ⁰C for 96 h in rotation mode. After the completion of synthesis the product was filtered, washed with distilled water, dried at 110 ⁰C and calcined at 550 ⁰C for 8 hours and MWW zeolite nuclei precursor was obtained.

### Example 19a

### Synthesis of mesoporous molecular sieve embedded with MWW structure, Na-MM-4MW22, without aluminium source

The synthesis of Na-MM-4MW22 was carried out by preparing of solutions A, B and C. Solution A was prepared by mixing 8.3 g fumed silica with 51.7 g distilled water with continuous stirring (r.m.p. 196) for 20 minutes. Solution B was prepared by adding 18.10 g of tetramethylammonium silicate to 11.7 g sodium silicate with continuous stirring (r.m.p 180) and the mixture was stirred for 20 minutes. Solution C was prepared by dissolving 26.3 g of tetradecyl trimethyl ammonium bromide in 174.3 g distilled water with vigorous stirring (r.m.p. 336) for 20 minutes. Solution B was added to Solution A slowly (in 15 min) with vigorous stirring (r. m. p. 320) after the addition of all of solution B the mixture was stirred for further 20 min. Solution C was added to mixture (A + B) slowly (20 min) with vigorous stirring (r. m. p. 336) after addition of solution C the mixture was further stirred for 20 minutes.

After that 4.22 g of MWW zeolite nuclei precursor prepared above was added to the gel solutions (A +B +C) under vigorous stirring (r.m.p. 340) for 20 min. The homogenisation of the dispersed MWW was carried out by further stirring (r.m.p. 340) of the gel for 35 minutes. After that gel was allowed to ripen for three hours with stirring (r.m.p 180) at ambient temperature. pH of the gel was controlled and the gel was introduced in Teflon cups which were then inserted in an autoclave. The synthesis was carried out for 96 h at 100 ⁰C.

After completion of the synthesis, the reactor was cooled for 30 min and mesoporous molecular sieve material embedded with MWW structure was mixed with distilled water, filtered and washed thoroughly with distilled water for 3 h. As synthesized Na-MM-4MW22 was dried at 110 ⁰C and calcined at 550 ⁰C using step calcinations procedure for 10 h in a muffle oven.

### Example 19b

### Preparation of H-MM-4MW22

10 g Na-MM-4MW22 (sodium form, prepared above) was ion-exchanged with 1 M ammonium nitrate or ammonium chloride aqueous solution for 24 h at ambient temperature. After ion-exchange the obtained NH₄-MM-4MW22 mesoporous molecular sieve material was washed thoroughly with distilled water, dried at 110 ⁰C for 12 hours and calcined at 450 ⁰C for four hours in a muffle oven using step calcinations procedure.

### Example 20

### Synthesis of mesoporous molecular sieve embedded with MWW structure, Na-MM-4MW22-2AI, using aluminium source

### Example 20a

### Synthesis of Na-MM-4MW22-2AI

The synthesis of Na-MM-4MW22-2AI was carried out by preparing solutions A, B and C. Solution A was prepared by mixing 8.3 g fumed silica with 51.7 g distilled water with continuous stirring (r.m.p. 196) for 20 minutes. Solution B was prepared by adding 18.10 g of tetramethylammonium silicate to 11.7 g sodium silicate with continuous stirring (r.m.p 180) and the mixture was stirred for 20 minutes. Solution C was prepared by dissolving 26.3 g of tetradecyl trimethyl ammonium bromide in 174.3 g distilled water with vigorous stirring (r.m.p. 336) for 20 minutes. Solution B was added to Solution A slowly (in 15 min) with vigorous stirring (r. m. p. 320) after the addition of solution B the mixture was stirred for further 20 min. Solution C was added to Solutions (A + B) slowly (20 min) with vigorous stirring (r. m. p. 336) and after the addition of all of solution C it was further stirred for 20 minutes.

4.2 g of MWW zeolite nuclei prepared above was added to the gel solutions (A +B +C) under vigorous stirring (r.m.p. 340) for 20 min. The homogenisation of the dispersed MWW was carried out by further vigorous stirring (r.m.p. 340) of the gel for 35 minutes. Then 2.3 g of aluminium isopropoxide was added and stirred for 20 min. After that gel was allowed to ripen for three hours with stirring (r.m.p 180) at ambient temperature. pH of the gel was controlled and gel was introduced in teflon cups which was then inserted in 300 ml autoclaves. The synthesis was carried out for 96 h at 100 ⁰C.

After completion of the synthesis, the reactor was quenched and mesoporous molecular sieve material embedded with MWW structure was filtered and washed thoroughly with distilled water for 3 h. As synthesized Na-MM-4MW22-2AI was dried at 110 ⁰C and calcined at 550 ⁰C using step calcinations procedure for 10 h.

### Example 20b

### Preparation of H-MM-4MW22-2AI

10 g Na-MM-4MW22-2AI (sodium form, prepared above) was ion-exchanged with 1 M ammonium nitrate or ammonium chloride aqueous solution for 24 h at ambient temperature. After ion-exchange the obtained NH₄-MM-4MW22-2AI mesoporous molecular sieve material was washed thoroughly with distilled water, dried at 110 ⁰C for 12 hours and calcined at 450 ⁰C for four hours in a muffle oven using step calcinations procedure.

The XRD pattern of the obtained H-MM-4MW22-2AI was similar to that of Na-MM-4MW22-2AI indicating that the aqueous treatment of the mesoporous material obtained by the present method and subsequent thermal treatment did not influence the stability of the structure.

### Example 21

### Synthesis of mesoporous molecular sieve embedded with MWW structure, Na-MM-4MW22-2AI-35, using aluminium source

### Example 21a

### Synthesis of Na-MM-4MW22-2AI-35

The synthesis of Na-MM-4MW22-2AI-35 was carried out by preparing of solutions A, B and C. Solution A was prepared by mixing 4.5 g of fumed silica with 51.7 g distilled water with continuous stirring (r.m.p. 196) for 20 minutes. Solution B was prepared by adding 18.10 g of tetramethylammonium silicate to 11.7 g sodium silicate with continuous stirring (r.m.p 180) and the obtained mixture was stirred for 20 minutes. Solution C was prepared by dissolving 26.3 g of tetradecyl trimethyl ammonium bromide in 174.3 g of distilled water with vigorous stirring (r.m.p. 336) for 20 minutes. Solution B was added to Solution A slowly (in 15 min) with vigorous stirring (r. m. p. 320) after addition of all of solution B the mixture was stirred for further 20 min. Solution C was added to Solutions (A + B) slowly (20 min) with vigorous stirring (r. m. p. 336) after the addition of solution C the mixture was further stirred for 20 minutes.

4.2 g of MWW zeolite nuclei prepared in example 19 was dispersed to the gel mixture (A +B +C) under vigorous stirring (r.m.p. 340) for 20 min. The homogenisation of the dispersed MWW was carried out by further stirring (r.m.p. 340) of the gel for 35 minutes. Then 2.3 g of aluminium isopropoxide was added to the mixture and stirred for 20 min. After that gel was allowed to ripen for three hours with stirring (r.m.p 180) at ambient temperature. pH of the gel was controlled and the gel was introduced in teflon cups which were then inserted in 300 ml autoclaves. The synthesis was carried out for 96 h at 100 ⁰C.

After completion of the synthesis, the reactor was quenched and the obtained mesoporous molecular sieve material embedded with MWW structure was mixed with distilled water, filtered and washed thoroughly with distilled water for 3 h. As synthesized Na-MM-4MW22-2AI-35 was dried at 110 ⁰C and calcined at 550 ⁰C in a muffle oven using step calcinations procedure for 10 h.

### Example 21b

### Preparation of H-MM-4MW22-2AI-35

10 g Na-MM-4MW22-2AI-35 (sodium form, prepared above) was ion-exchanged with 1 M ammonium nitrate aqueous solution for 24 h at ambient temperature. After ion-exchange the obtained NH₄-MM-4MW22-2AI-35 mesoporous molecular sieve material was washed thoroughly with distilled water, dried at 110 ⁰C for 12 hours and calcined at 450 ⁰C for four hours in a muffle oven using step calcinations procedure.

The XRD pattern of the obtained H-MM-4MW22-2AI-35 was similar to that of Na-MM-4MW22-2AI-35 indicating that the aqueous treatment of the mesoporous material obtained by the present method and subsequent thermal treatment did not influence the stability of the structure.

### Example 22

### Preparation of platinum modified H-MM-4MW22-2AI

5 g of H-MM-4MW22-2AI was loaded with 2 wt % Pt using impregnation method. 2 wt % Pt impregnation was performed in a rotary evaporator at 80 ⁰C for 24 h using aqueous solution of hexachloroplatinic acid. The 2 wt % impregnated H-MM-4MW22-2AI was dried at 100 °C and calcined at 450 °C. The XRD pattern of Pt-H-MM-4MW22-2AI presented was similar to those of parent Na-MM-4MW22-2AI indicating hydrothermal stability of the mesoporous molecular sieve embedded with MWW structure obtained by the present method.

### Example 23

### Preparation of platinum modified H-MM-4MW22-2AI-35

5 g of H-MM-4MW22-2AI-35 was loaded with 2 wt % Pt using impregnation method. 2 wt % Pt impregnation was performed in a rotary evaporator at 80 ⁰C for 24 h using aqueous solution of hexachloroplatinic acid. The 2 wt % impregnated H-MM-4MW22-2AI-35 was dried at 100 ⁰C and calcined at 450 ⁰C. The XRD pattern of Pt-H-MM-4MW22-2AI-35 was similar to that of parent Na-MM-4MW22-2AI-35 indicating the hydrothermal stability of the mesoporous molecular sieve embedded with MWW structure obtained by the present method.

### Examples 24- 28

### Mesoporous molecular sieve embedded with MOR zeolite structure

### Preparation of MOR zeolite nuclei

Two solutions A and B were made for preparation of MOR zeolite nuclei precursor. Solution A was prepared by adding 37.8 g of Ludox AS 30 to 6.7 g of piperidine and stirred for 15 min. Solution B was prepared by adding 44 ml of distilled water to 4.6 g of sodium hydroxide and stirred for 10 minutes and then 5.9 g of aluminium sulphate was added and further stirred for 15 minutes. Solution B was added to Solution A slowly and with vigorous stirring for 15 minutes. The gel was introduced in Teflon cups and inserted in 300 ml autoclave. The synthesis was carried out at 200 °C for 48 h in rotation mode. After completion of the synthesis the product was filtered, washed with distilled water, dried at 110 °C and calcined at 550 °C for 10 hours and MOR zeolite nuclei precursor was obtained.

### Example 24a

### Synthesis of mesoporous molecular sieve embedded with MOR structure, Na-MM-MO-4MO-96 h, without aluminium source

The synthesis of Na-MM-MO-4MO-96 h was carried out by preparing of solutions A, B and C. Solution A was prepared by mixing 8.3 g fumed silica with 51.7 g distilled water with continuous stirring (r.m.p. 196) for 20 minutes. Solution B was prepared by adding 18.10 g of tetramethylammonium silicate to 11.7 g sodium silicate with continuous stirring (r.m.p 180) and the mixture was stirred for 20 minutes. Solution C was prepared by dissolving 26.3 g of tetradecyl trimethyl ammonium bromide (Fluka) in 174.3 g distilled water with vigorous stirring (r.m.p. 336) for 20 minutes. Solution B was added to Solution A slowly (in 15 min) with vigorous stirring (r. m. p. 320) after addition of all of solution B the mixture was stirred for further 20 min. Solution C was added to the mixture A + B slowly (20 min) with vigorous stirring (r. m. p. 336) after addition of all of solution C the mixture was further stirred for 20 minutes.

After that 3.7 g of MOR zeolite nuclei precursor prepared above was added to the gel mixture (A +B +C) under vigorous stirring (r.m.p. 350) for 20 min. The homogenisation of the dispersed MOR structure was carried out by further stirring (r.m.p. 350) of the gel for 30 minutes. After that gel was allowed to ripen for three hours with stirring (r.m.p 180) at ambient temperature. pH of the gel was controlled and gel was introduced in a teflon cup which was then inserted in a 300 autoclave. The synthesis was carried out for 96 h at 100 °C.

After completion of the synthesis, the reactor was quenched and mesoporous molecular sieve material embedded with MOR structure was mixed with distilled water, filtered and washed thoroughly with distilled water for 3 h. As synthesized Na-MM-MO-4MO-96 h was dried at 110 ⁰C and calcined at 550 °C using step calcinations procedure for 10 h.

### Example 24b

### Preparation of H-MM-MO-4MO-96 h

10 g Na-MM-MO-4MO-96 h (sodium form, prepared above) was ion-exchanged with 1 M ammonium nitrate or ammonium chloride aqueous solution for 24 h at ambient temperature. After ion-exchange the obtained NH₄-MM-MO-4MO-96 h mesoporous molecular sieve material was washed thoroughly with distilled water, dried at 110 °C for 12 hours and calcined at 450 ⁰C for four hours in a muffle oven using step calcinations procedure.

The XRD pattern of the obtained H-MM-MO-4MO-96 h was similar to that of Na-MM-MO-4MO-96h indicating that the aqueous treatment of the mesoporous material obtained by the present method and subsequent thermal treatment did not influence the stability of the structure.

### Example 25

### Synthesis of mesoporous molecular sieve embedded with MOR structure, Na-MM-MO-4MO-96 h-2AI, using aluminium source

### Example 25a

### Synthesis of Na-MM-MO-4MO-2AI

The synthesis of Na-MM-MO-4MO-96 h-2AI was carried out by preparing of solutions A, B and C. Solution A was prepared by mixing 8.3 g fumed silica with 51.7 g distilled water with continuous stirring (r.m.p. 196) for 20 minutes. Solution B was prepared by adding 18.10 g of tetramethylammonium silicate to 11.7 g sodium silicate with continuous stirring (r.m.p 180) and the obtained mixture was stirred for 20 minutes. Solution C was prepared by dissolving 26.34 g of tetradecyl trimethyl ammonium bromide in 174.3 g distilled water with vigorous stirring (r.m.p. 336) for 20 minutes. Solution B was added to Solution A slowly (in 15 min) with vigorous stirring (r. m. p. 320) and after the addition of all of solution B the mixture was stirred for further 20 min. Solution C was added to mixture A + B slowly (20 min) with vigorous stirring (r. m. p. 336) after addition of all of solution C it was further stirred for 20 minutes.

3.7 g of MOR zeolite nuclei precursor prepared in example 23 was added to the gel mixtures (A +B +C) under vigorous stirring (r.m.p. 350) for 25 min. and then 1.9 g of aluminium isopropoxide was added to the gel under vigorous stirring (r.m.p. 350) and the gel was stirred for 30 minutes. After that gel was allowed to ripen for three hours with stirring (r.m.p 180) at ambient temperature. pH of the gel was controlled and gel was introduced in teflon cups which were then inserted in 300 ml autoclaves. The synthesis was carried out for 96 h at 100 °C.

After completion of the synthesis, the reactor was quenched for 30 min and mesoporous molecular sieve material embedded with MOR structure was mixed with distilled water, filtered and washed thoroughly with distilled water for 3 h. As synthesized Na-MM-MO-4MO-96h-2AI was dried at 110 ⁰C and calcined at 550 °C using step calcinations procedure for 10 h.

### Example 25b

### Preparation of H-MM-MO-4MO-96h-2AI

10 g Na-MM-MO-4MO-96 h-2AI (sodium form, prepared above) was ion-exchanged with 1 M ammonium nitrate or ammonium chloride aqueous solution for 24 h at ambient temperature. After ion-exchange the obtained NH₄-MM-MO-4MO-96h-2AI mesoporous molecular sieve material was washed thoroughly with distilled water, dried at 110 °C for 12 hours and calcined at 450 °C for four hours in a muffle oven using step calcinations procedure.

The XRD pattern of the obtained H-MM-MO-4MO-96 h-2AI was similar to that of Na-MM-4MO-96h-2AI indicating that the aqueous treatment of the mesoporous material obtained by the present method and subsequent thermal treatment did not influence the stability of the structure.

### Example 26

### Synthesis of mesoporous molecular sieve embedded with MOR structure, Na-MM-MO-4MO-2AI-35, using aluminium source

### Example 26a

### Synthesis of Na-MM-MO-4MO-2AI-35

The synthesis of Na-MM-MO-4MO-96 h-2AI-35 was carried out by preparing of solutions A, B and C. Solution A was prepared by mixing 4.4 g of fumed silica with 51.7 g distilled water with continuous stirring (r.m.p. 196) for 20 minutes. Solution B was prepared by adding 18.10 g of tetramethylammonium silicate to 11.7 g sodium silicate with continuous stirring (r.m.p 180) and the mixture was stirred for 20 minutes. Solution C was prepared by dissolving 26.3 g of tetradecyl trimethyl ammonium bromide in 174.3 g distilled water with vigorous stirring (r.m.p. 336) for 20 minutes. Solution B was added to Solution A slowly (in 15 min) with vigorous stirring (r. m. p. 320) after addition of all of solution B the mixture was stirred for further 20 min. Solution C was added to mixture (A + B) slowly (20 min) with vigorous stirring (r. m. p. 336) and after addition of all of solution C the mixture was further stirred for 20 minutes.

3.7 g of MOR zeolite nuclei precursor prepared in example 45 was added to the gel mixture (A +B +C) under vigorous stirring (r.m.p. 320) for 25 min. Then 1.9 g of aluminium isopropoxide was added and stirred for 20 min. After that gel was allowed to ripen for three hours with stirring (r.m.p 180) at ambient temperature. pH of the gel was controlled and the gel was introduced in teflon cups which were then inserted in 300 ml autoclave. The synthesis was carried out for 96 h at 100 °C.

After completion of the synthesis, the reactor was quenched for 30 min and mesoporous molecular sieve material embedded with MOR structure was mixed with distilled water, filtered and washed thoroughly with distilled water for 3 h. As synthesized Na-MM-MO-4MO-96 h-2AI-35 was dried at 110 °C and calcined at 550 ⁰C using step calcinations procedure for 10 h.

### Example 26b

### Preparation of H-MM-MO-4MO-96h-2AI-35

10 g Na-MM-MO-4MO-2AI-35 (sodium form, prepared above) was ion-exchanged with 1 M ammonium nitrate or ammonium chloride aqueous solution for 24 h at ambient temperature. After ion-exchange the obtained NH₄-MM-MO-4MO-2AI-35 mesoporous molecular sieve material was washed thoroughly with distilled water, dried at 110 °C for 12 hours and calcined at 450 °C for four hours in a muffle oven using step calcinations procedure.

### Example 27

### Preparation of Platinum modified H-MM-MO-4MO-96 h-2AI-35

5 g of H-MM-MO-4MO-96 h-2AI-35 was loaded with 2 wt % Pt using impregnation method. 2 wt % Pt impregnation was performed in a rotary evaporator at 80 ⁰C for 24 h using aqueous solution of hexachloroplatinic acid. The 2 wt % impregnated MM-MO-4MO-96 h-2AI-35 was dried at 100 ⁰C and calcined at 450 ⁰C. The XRD patterns of Pt-H-MM-MO-4MO-96 h-2AI were similar to those of parent Na-MM-MO-4MO-96 h-2AI-35 indicating hydrothermal stability of the mesoporous molecular sieve embedded with MOR structure obtained by the present method.

### Example 28

### Preparation of platinum modified H-MM-MO-4MO-96 h-2AI-35

5 g of H-MM-MO-4MO-2AI-35 was loaded with 2 wt % Pt using impregnation method. 2 wt % Pt impregnation was performed in a rotary evaporator at 80 ⁰C for 24 h using aqueous solution of hexachloroplatinic acid. The 2 wt % impregnated HMM-MO-4MO-2AI-35 was dried at 100 ⁰C and calcined at 450 ⁰C. The XRD patterns of Pt-H-MM-MO-4MO-96 h-2AI-35 were similar to those of parent Na-MM-MO-4MO-96 h-2AI-35 indicating hydrothermal stability of the mesoporous molecular sieve embedded with MOR structure obtained by the present method.

### Example 29

### Thermal stability test

The thermal stability test was performed by heating the materials obtained by the method according to the invention at temperatures 700 °C, 800 °C, 900 °C, and 1000 °C in air for 24 hours. After this treatment the materials were analysed by BET and XRD. An example for XRD diagram at 1000 °C treated samples is given in Fig. 5. No differences in the structure of the materials could be detected with BET or XRD

### Example 30

### Mechanical stability test

Mechanical stability test of the materials obtained by the method according to the invention was performed by pressing the powder of the material with a pressure of 20 000 Newton. The tablets formed were crushed and sieved in different particle sizes. The XRD and BET measurements of different fractions of the sieved powder were analysed by XRD and BET. No differences in the XRD diagrams or in BET surface areas and pore size distributions could be observed. The results are presented in the following table 4, giving examples of N₂-adsorption measurement results of mechanical stability tests.

**Table 4.**

| **Surface area and porosity by N₂ adsorption** | **BET area (m²/g)** | **BJH area (m²/g)** | **BET pore volume (cm³/g)** | **BJH pore volume (cm³/g)** | **BET pore diameter (Å)** | **BJH pore diameter (Å)** |
|---|---|---|---|---|---|---|
| H-MM5-96h-4ZS 0.6-0.85 | 775 | 731 | 0.594 | 0.513 | 32 | 28 |
| H-MM5-96h-4ZS <0.007 | 748 | 682 | 0.563 | 0.489 | 31 | 29 |

### Example 31

### Reproducibility of the methods

The same procedure for the manufacture of the material according to the invention (example 8) was repeated in different scales. The batches showed very similar properties as shown in table 5, presenting results of reproducibility test.

**Table 5.**

| | **Si (wt%)** | **Al(wt%)** | **Si/Al** | **MCM-41 a₀ (Å)** | **MFI a₀ (Å)** |
|---|---|---|---|---|---|
| Example 8 | 42.3 | 3.9 | 10.4 | 38 | 19.72 |
| Example 8 (10x larger scale) | 42.7 | 3.8 | 10.9 | 36 | 19.73 |

### Examples 32-41

### Oligomerization of 1-decene with materials obtained by the method according to the invention as catalysts

Oligomerization of 1-decene with materials obtained by the method according to the invention as catalysts showed high activity, low deactivation and regenerability of the catalysts obtained by the method according to the invention. Catalytic materials obtained by the method according to the invention and comparative catalysts according to the state of the art were tested in oligomerization of 1 -decene. The tests were carried out in a batch reactor under stirring. Reaction temperature was 200 °C. Reaction time was 24 h. The pressure in the reactor was about 20 bar.

The reaction products were analyzed by GC and by GC-distillation, and the peaks were identified based on the carbon number of molecule. The molecules with carbon number above 20 were identified as lubricant components in GC-analysis. The molecules boiled above 343 °C were identified as lubricant molecules in GC-distillation.

The catalysts used in the tests were regenerated in a muffle oven in air at 540 °C temperature.

The results from the 1-decene oligomerization reaction tests are summarized in following table 6.

**Table 6.**

| **Example** | **Catalyst** | **Conversion, %** | **Selectivity to lubricant, %** | **Yield to lubricant, %** |
|---|---|---|---|---|
| 32 | ZSM-5 (ex. 1) | 20 | 3 | 0.5 |
| 33 | MSA-1 (ex. 2) | 0.5 | 87 | 0.4 |
| 34 | MSA-3 (ex. 4) | 1 | 87 | 0.9 |
| 35 | MCM-41 (ex. 5) | 45 | 97 | 29 |
| 36 | H-MM5-96h4ZS2Al35 (ex. 9) | 71 | 97 | 69 |
| 37 | H-MMBE-96h-4B-2Al (ex. 12) | 78 | 94 | 73 |
| 38 | H-MMBE-96-4B-2Al35 (ex.13) | 80 | 93 | 74 |
| 39 | H-MM5-96h4ZS2Al35 Regen. | 70 | 98 | 69 |
| 40 | H-MMBE-96h-4B-2Al Regen. | 76 | 96 | 73 |
| 41 | H-MMBE-96-4B-2Al35 Regen. | 79 | 93 | 72 |

| | | | | |
|---|---|---|---|---|
| Regen = regenerated | | | | |

### Examples 42 and 43

### Isobutene reaction with materials obtained by the method according to the invention as catalysts

Isobutene reaction tests were performed with materials obtained by the method according to the invention as catalysts, showing the high activity and low deactivation of the catalysts obtained by the method according to the invention. The catalysts were tested in a fixed bed reactor at a reaction temperature of 100°C under 20 bar with WHSV 20. High activity and no deactivation of the catalysts were observed. As example isobutene reaction the catalyst of example 8 is compared with the comparative catalyst (example 1) in Figure 7 in isobutene dimerization.

### Examples 44-47

### Paraffin isomerization tests with materials obtained by the method according to the invention as catalysts

The purpose of n-butane isomerization test reaction was to confirm the chemical nature of the interaction in the mesoporous molecular sieve embedded with MFI structure obtained by the method according to the invention, formation of strong Bronsted acid sites and hydrothermal stability of material obtained by the present method. n-Butane isomerization was used as a test reaction for the evaluation of acidity of catalysts. n-Butane isomerization was carried out over the proton form of the present mesoporous molecular sieve catalysts to evaluate the acidic properties. It was observed that the H-form (H-MM5-96h-4ZS-2AI-35) catalyst with lowest Si/Al ratio showed highest conversion of n-butane, clearly indicating the formation of strong Brønstedt acid sites and formation of a present mesoporous molecular sieve embedded with a MFI structure with true chemical bonding.

The regeneration of the H-form and Pt-H-MM5 catalysts was carried out in presence of air at 450 °C for two hours. The purpose of the regeneration was to evaluate whether it was possible to regain the catalytic activity, further more also to evaluate the hydrothermal stability of the catalyst during the regeneration of the catalyst since water is produced during the regeneration process. It was verified that both the H-form and Pt modified catalysts almost completely retained their catalytic activity, confirming hydrothermal stability of the structure.

Isomerization of n-butane to isobutane was investigated over the proton form catalyst and 2 wt % Pt-H-MM5-96h-4ZS-2AI catalysts in a fixed-bed micro-reactor made of quartz. The experiments were carried out near atmospheric pressure and the amounts of the catalyst used were 0.3 - 1.0 g. The reactant n-butane was fed into the reactor using hydrogen as a carrier gas. The product analysing was carried out on-line using a gas chromatograph equipped with a FI detector and a capillary column. The results from the n-butane isomerization test reactions are provided in Table 7, presenting n-Butane isomerization at a temperature of 450 °C, WHSV 1.23 h⁻¹, n-butane / hydrogen ratio 1:1.

**Table 7. Conversion of n-butane**

| **Example no** | **Catalyst** | **Conversion (wt %)** |
|---|---|---|
| 44 | H-NM5-96h-4ZS-2AI | 40 |
| 45 | H-MM5-96h-4ZS-2AI-35 | 70 |
| 46 | Pt-H-MM5-96h-4ZS-2AI-35-C-Fesh | 87 |
| 47 | Pt-H-MM5-96h-4ZS-2AI-35-C-Reg | 85 |

### Examples 48 and 49

### 1-Butene isomerization tests with materials obtained by the method according to the invention as catalysts

The purpose of 1-butene isomerization test reaction was to confirm the chemical interaction in the mesoporous molecular sieve embedded with a MFI structure obtained by the method according to the invention, formation of the strong Bronsted acid sites and hydrothermal stability of the material obtained by the present method.

1-butene isomerization was also used as a test reaction for the investigation of isomerization of 1-butene to iso-butene. Further more the aim was to study the possibility of regeneration of used catalyst, and to evaluate if the catalyst retains its catalytic activity after regeneration. It was found that the used catalyst, after the 1-butene isomerization could be regenerated. Further more the regenerated catalyst exhibited almost the same conversion (97.2 mol %) of 1-butene as the corresponding fresh catalyst (97 mol %), indicating also the hydrothermal stability of the catalyst.

The isomerization of 1-butene to isobutene was investigated over proton form H-MM5-96h-4ZS-2AI catalyst in a fixed-bed micro-reactor made of quartz. The experiments were carried out near atmospheric pressure at a temperature of 350 °C with WHSV 10 h⁻¹. The reactant 1-butene was fed into the reactor using nitrogen as a carrier gas with a ratio 1:1. The product analysis was carried out on-line using a gas chromatograph equipped with a FI detector and a capillary column. A condenser was placed after the GC in order to facilitate liquid product sampling of the heavier compounds. The first sample was taken after 10 minutes on stream (TOS). The first 10 samples were taken at 1 h intervals and the subsequent samples every 3 h.

### Example 50

### Ring Opening test with material obtained by the method according to the invention as catalyst

The activity and selectivity of the catalyst obtained by the method according to the invention in decalin ring opening reaction was tested in a 50 ml autoclave at 250 °C in 20 bar hydrogen pressure. Decalin (10 ml ~9.0 g) was added to the reactor containing 1 g of at 250 °C reduced catalyst in room temperature. The pressure was increased with hydrogen to 10 bar. Then the reactor was placed in an oil bath at 250 °C. When the temperature of the reactor reached 250 °C, the hydrogen pressure was adjusted to 20 bar. The reaction time was five hours. Then the reactor was cooled rapidly to -10 °C. The reactor was weighted after the cooling. The pressure in autoclave was released. The product containing the catalyst was taken to a sample vessel and a GC-sample was taken through a needle with a filter. The conversion of decalin was 81 % and the selectivity to ring opening products 32 % with the catalyst obtained by the method according to the invention, prepared according to example 16.

### Examples 51 and 52

### Hydrocracking tests with material obtained by the method according to the invention as catalyst

The activity and selectivity of the catalyst obtained by the method according to the invention (catalyst of example 17) in hydrocracking reaction was tested in an autoclave at 300°C (example 51) and 350°C (example 52) under 30 bar hydrogen pressure. Paraffin mixture (about 80 g) was added to the reactor containing 2 g of at 400 °C reduced catalyst at room temperature. The pressure was increased with hydrogen to 30 bar. When the temperature of the reactor reached 300 °C (example 51) or 350°C (example 52), the hydrogen pressure was adjusted to 30 bar. The reaction time was 65 hours. The reactor was weighted after the cooling. The pressure in autoclave was released. The products were analysed by GC. The conversion of paraffins was 60 % (example 51) and 65% (example 52) and the selectivity to the cracking products in both cases was 100 %.

## Claims

1. A method for the manufacture of a catalytic material which catalytic material is a zeolite embedded within a mesoporous molecular sieve, wherein the zeolite is selected from MFI, MTT, TON, AEF, MWW, FER, BEA, FAU, MOR zeolites, **characterized in that** the method comprises the steps:
a) preparing of zeolite nuclei from a silicon source and an aluminium source and structure directing agent, and optionally removing the structure directing agent with a step calcination procedure;
b) preparing of mesoporous molecular sieve gel mixture from a silicon source, an optional aluminium source, and surfactant;
c) introducing the zeolite nuclei prepared in step a) to the mesoporous molecular sieve gel mixture obtained in step b), and homogenising and dispersing in the molecular sieve gel the zeolite nuclei, and introducing an additional aluminium source to the obtained mixture;.
d) performing gel ripening of the mixture of step c) under stirring;
e) carrying out hydrothermal synthesis of the mixture of step d) by maintaining the mixture under sufficient conditions including a temperature of from 100 °C to 200 °C under static or dynamic mode of stirring until crystals are formed;
f) recovering the crystals;
g) washing of the solid product;
h) drying of the solid product, and
i) removing the surfactant partly or totally with a step calcination procedure and optionally the structure directing agent if it was not removed in step a), whereby catalytic material is obtained.

2. The method according to claim 1 for the manufacture of a catalytic material which catalytic material is a zeolite embedded within a mesoporous molecular sieve, wherein the zeolite is selected from MFI, MTT, TON, AEF, MWW, FER, BEA, FAU, MOR zeolites, **characterized in that** the silicon source in step a) is selected from silicon oxides, preferably from colloidal silica, solid silica and fumed silica.

3. The method according to claim 1 or 2 for the manufacture of a catalytic material which catalytic material is a zeolite embedded within a mesoporous molecular sieve, wherein the zeolite is selected from MFI, MTT, TON, AEF, MWW, FER, BEA, FAU, MOR zeolites, **characterized in that** the silicon source or sources in step b) is selected from silicon compounds having an organic group and preferably the silicon source having an organic group is tetraethoxy silane, tetramethylammonium silicate or tetraethylammonium silicate.

4. The method according to claim 1 or 2 for the manufacture of a catalytic material which catalytic material is a zeolite embedded within a mesoporous molecular sieve, wherein the zeolite is selected from MFI, MTT, TON, AEF, MWW, FER, BEA, FAU, MOR zeolites, **characterized in that** the silicon source or sources in step b) is selected from inorganic silicon sources and preferably the inorganic silicon source is sodium silicate, water glass, colloidal silica, solid silica or fumed silica.

5. The method according to any one of claims 1-4 for the manufacture of a catalytic material which catalytic material is a zeolite embedded within a mesoporous molecular sieve, wherein the zeolite is selected from MFI, MTT, TON, AEF, MWW, FER, BEA, FAU, MOR zeolites, **characterized in that** the aluminium source in step a) and b) is selected from aluminium sulphate (Al₂(SO₄)₃.18H₂O), hydrated aluminium hydroxides, aluminates, aluminium isoproxide and alumina.

6. The method according to any one of claims 1 - 5 for the manufacture of a catalytic material which catalytic material is a zeolite embedded within a mesoporous molecular sieve, wherein the zeolite is selected from MFI, MTT, TON, AEF, MWW, FER, BEA, FAU, MOR zeolites, **characterized in that** the surfactant is selected from alkyltrimethyl ammonium halide compounds with the general formula CₙH₂ₙ₊₁ (CH₃)₃∗NX, where n = 12 to 18, X= Cl, Br, and preferably the surfactant is n-hexadecyltrimethyl ammonium bromide, n-hexadecyltrimethyl ammonium chloride, cetyltrimethylammonium bromide and cetyltriethylammonium bromide.

7. The method according to any one of claims 1 - 6 for the manufacture of a catalytic material which catalytic material is a zeolite embedded within a mesoporous molecular sieve, wherein the zeolite is selected from MFI, MTT, TON, AEF, MWW, FER, BEA, FAU, MOR zeolites, **characterized in that** the additional aluminium source is selected from aluminium alkoxides, preferably aluminium isopropoxide.

## Patentansprüche

1. Verfahren für die Herstellung eines katalytischen Materials, wobei das katalytische Material ein innerhalb eines mesoporösen Molekularsiebs eingebetteter Zeolith ist, wobei der Zeolith ausgewählt wird aus MFI-, MTT-, TON-, AEF-, MWW-, FER-, BEA-, FAU-, MOR-Zeolithen, **dadurch gekennzeichnet, dass** das Verfahren die Schritte umfasst:
a) Zubereiten von Zeolithkernen aus einer Siliciumquelle und einer Aluminiumquelle und einem strukturgebenden Mittel, und optional Entfernen des strukturgebenden Mittels mit einem schrittweisen Kalzinierungsverfahren;
b) Zubereiten einer mesoporösen Molekularsieb-Gel-Mischung aus einer Siliciumquelle, einer optionalen Aluminiumquelle und einem Tensid;
c) Einbringen der in Schritt a) zubereiteten Zeolithkerne in die in Schritt b) erhaltene mesoporöse Molekularsieb-Gel-Mischung und Homogenisieren und Dispergieren der Zeolithkerne in dem Molekularsieb-Gel, und Einbringen einer zusätzlichen Aluminiumquelle in die erhaltene Mischung;
d) Durchführen einer Gelreifung des Gemischs aus Schritt c) unter Rühren;
e) Durchführen einer hydrothermalen Synthese des Gemischs des Schritts d) durch Halten des Gemisches unter ausreichenden Bedingungen einschließlich einer Temperatur von 100°C bis 200°C unter statischem oder dynamischem Rühren, bis sich Kristalle bilden;
f) Gewinnen der Kristalle;
g) Waschen des festen Produkts;
h) Trocknen des festen Produkts, und
i) teilweises oder vollständiges Entfernen des Tensids mit einem Kalzinierungsverfahren, und optional des strukturgebenden Mittels, wenn es nicht in Schritt a) entfernt wurde, wodurch ein katalytisches Material erhalten wird.

2. Verfahren nach Anspruch 1 für die Herstellung eines katalytischen Materials, wobei das katalytische Material ein innerhalb eines mesoporösen Molekularsiebs eingebetteter Zeolith ist, wobei der Zeolith ausgewählt wird aus MFI-, MTT-, TON-, AEF-, MWW-, FER-, BEA-, FAU-, MOR-Zeolithen, **dadurch gekennzeichnet, dass** die Siliciumquelle in a) ausgewählt wird aus Siliciumoxiden, bevorzugt aus kolloidalem Siliciumdioxid, festem Siliciumdioxid und pyrogenem Siliciumdioxid.

3. Verfahren nach Anspruch 1 oder 2 für die Herstellung eines katalytischen Materials, wobei das katalytische Material ein innerhalb eines mesoporösen Molekularsiebs eingebetteter Zeolith ist, wobei der Zeolith ausgewählt wird aus MFI-, MTT-, TON-, AEF-, MWW-, FER-, BEA-, FAU-, MOR-Zeolithen, **dadurch gekennzeichnet, dass** die Siliciumquelle oder -quellen in Schritt b) ausgewählt wird aus Siliciumverbindungen mit einer organischen Gruppe und bevorzugt ist die Siliciumquelle mit einer organischen Gruppe Tetraethoxysilan, Tetramethylammoniumsilicat oder Tetraethylammoniumsilicat.

4. Verfahren nach Anspruch 1 oder 2 für die Herstellung eines katalytischen Materials, wobei das katalytische Material ein innerhalb eines mesoporösen Molekularsiebs eingebetteter Zeolith ist, wobei der Zeolith ausgewählt wird aus MFI-, MTT-, TON-, AEF-, MWW-, FER-, BEA-, FAU-, MOR-Zeolithen, **dadurch gekennzeichnet, dass** die Siliciumquelle oder -quellen in Schritt b) ausgewählt wird aus anorganischen Siliciumquellen und bevorzugt ist die anorganische Siliciumquelle Natriumsilicat, Wasserglas, kolloidales Siliciumdioxid, festes Siliciumdioxid oder pyrogenes Siliciumdioxid.

5. Verfahren nach einem der Ansprüche 1 - 4 für die Herstellung eines katalytischen Materials, wobei das katalytische Material ein innerhalb eines mesoporösen Molekularsiebs eingebetteter Zeolith ist, wobei der Zeolith ausgewählt wird aus MFI-, MTT-, TON-, AEF-, MWW-, FER-, BEA-, FAU-, MOR-Zeolithen, **dadurch gekennzeichnet, dass** die Aluminiumquelle in Schritt a) und b) ausgewählt wird aus Aluminiumsulfat (Al₂(SO₄)₃.18H₂O), hydrierten Aluminiumhydroxiden, Aluminaten, Aluminiumisoproproxid und Aluminiumoxid.

6. Verfahren nach einem der Ansprüche 1- 5 für die Herstellung eines katalytischen Materials, wobei das katalytische Material ein innerhalb eines mesoporösen Molekularsiebs eingebetteter Zeolith ist, wobei der Zeolith ausgewählt wird aus MFI-, MTT-, TON-, AEF-, MWW-, FER-, BEA-, FAU-, MOR-Zeolithen, **dadurch gekennzeichnet, dass** das Tensid ausgewählt wird aus Alkyltrimethylammoniumhalidverbindungen mit der allgemeinen Formel CₙH₂ₙ₊₁(CH₃)₃∗NX, wobei n=12 bis 18, X = Cl, Br, und bevorzugt ist das Tensid n-Hexadecyltrimethylammoniumbromid, n-Hexadecyltrimethylammoniumchlorid, Cetyltrimethylammoniumbromid und Cetyltriethylammoniumbromid.

7. Verfahren nach einem der Ansprüche 1 - 6 für die Herstellung eines katalytischen Materials, wobei das katalytische Material ein innerhalb eines mesoporösen Molekularsiebs eingebetteter Zeolith ist, wobei der Zeolith ausgewählt wird aus MFI-, MTT-, TON-, AEF-, MWW-, FER-, BEA-, FAU-, MOR-Zeolithen, **dadurch gekennzeichnet, dass** die zusätzliche Aluminiumquelle ausgewählt wird aus Aluminiumalkoxiden, bevorzugt Aluminiumisopropoxid.

## Revendications

1. Procédé de fabrication d'un matériau catalytique, lequel matériau catalytique est une zéolithe intégrée dans un tamis moléculaire mésoporeux, dans lequel la zéolithe est choisie parmi MFI, MTT, TON, AEF, MWW, FER, BEAU, FAU, et zéolithes MOR, **caractérisé en ce que** le procédé comprend les étapes suivantes :
a) préparation de noyaux de zéolite à partir d'une source de silicium et d'une source d'aluminium et d'un agent directeur de structure, et éventuellement élimination de l'agent directeur de structure au moyen d'une procédure de calcination par étapes ;
b) préparation d'un mélange de gel de tamis moléculaire mésoporeux à partir d'une source de silicium, d'une source d'aluminium facultative et d'un tensioactif ;
c) introduire les noyaux de zéolithe préparés à l'étape a) dans le mélange de gel de tamis moléculaire mésoporeux obtenu à l'étape b), et homogénéiser et disperser dans le gel de tamis moléculaire les noyaux de zéolithe, et introduire une source d'aluminium additionnelle au mélange obtenu ;
d) réalisation d'un mûrissement sur gel du mélange de l'étape c) sous agitation ;
e) réaliser la synthèse hydrothermale du mélange de l'étape d) en maintenant le mélange dans des conditions suffisantes dont une température comprise entre 100°C et 200°C sous un mode d'agitation statique ou dynamique jusqu'à formation de cristaux ;
f) récupérer les cristaux ;
g) lavage du produit solide ;
h) séchage du produit solide, et
i) élimination partielle ou totale du tensioactif au moyen d'une procédure de calcination par étapes et éventuellement de l'agent structurant s'il n'avait pas été éliminé à l'étape a), de sorte que du matériau catalytique est obtenu.

2. Procédé selon la revendication 1 pour la fabrication d'un matériau catalytique, lequel matériau catalytique est une zéolithe intégrée dans un tamis moléculaire mésoporeux, dans lequel la zéolithe est choisie parmi MFI, MTT, TON, AEF, MWW, FER, BEAU, FAU, et zéolithes MOR, **caractérisé en ce que** la source de silicium à l'étape a) est choisie parmi les oxydes de silicium, de préférence parmi la silice colloïdale, la silice solide et la silice pyrogénée.

3. Procédé selon la revendication 1 ou 2 pour la fabrication d'un matériau catalytique, lequel matériau catalytique est une zéolithe intégrée dans un tamis moléculaire mésoporeux, dans lequel la zéolithe est choisie parmi MFI, MTT, TON, AEF, MWW, FER, BEAU, FAU, et zéolithes MOR, **caractérisé en ce que** la ou les sources de silicium à l'étape b) sont choisies parmi des composés de silicium à groupement organique et de préférence la source de silicium ayant un groupe organique est le tétraéthoxysilane, le silicate de tétraméthylammonium ou le silicate de tétraéthylammonium,

4. Procédé selon la revendication 1 ou 2 pour la fabrication d'un matériau catalytique, lequel matériau catalytique est une zéolithe intégrée dans un tamis moléculaire mésoporeux, dans lequel la zéolithe est choisie parmi MFI, MTT, TON, AEF, MWW, FER, BEAU, FAU, et zéolithes MOR, **caractérisé en ce que** la ou les sources de silicium à l'étape b) sont choisies parmi des sources de silicium inorganiques et de préférence la source de silicium inorganique est du silicate de sodium, du verre soluble, de la silice colloïdale, de la silice solide ou de la silice pyrogénée.

5. Procédé selon l'une quelconque des revendications 1 à 4 pour la fabrication d'un matériau catalytique, lequel matériau catalytique est une zéolithe intégrée dans un tamis moléculaire mésoporeux, dans lequel la zéolithe est choisie parmi MFI, MTT, TON, AEF, MWW, FER, BEAU, FAU, et zéolithes MOR, **caractérisé en ce que** la source d'aluminium dans les étapes a) et b) est choisie parmi le sulfate d'aluminium (Al₂(SO₄)₃·18H₂O), les hydroxydes d'aluminium hydraté, les aluminates, l'isopropoxyde d'aluminium et l'alumine.

6. Procédé selon l'une quelconque des revendications 1 à 5 pour la fabrication d'un matériau catalytique, lequel matériau catalytique est une zéolithe intégrée dans un tamis moléculaire mésoporeux, dans lequel la zéolithe est choisie parmi MFI, MTT, TON, AEF, MWW, FER, BEAU, FAU, et zéolithes MOR, **caractérisé en ce que** le tensioactif est choisi parmi les composés halogénures d'alkyltriméthylammonium de formule générale CₙH2ₙ₊₁(CH₃)_{3*}NX, où n vaut de 12 à 18, X est Cl ou Br, et, de préférence, le tensioactif est le bromure de n-hexadécyltriméthylammonium, le chlorure de n-hexadécyltriméthylammonium, le bromure de cétyltriméthylammonium et le bromure de cétyltriéthylammonium.

7. Procédé selon l'une quelconque des revendications 1 à 6 pour la fabrication d'un matériau catalytique, lequel matériau catalytique est une zéolithe intégrée dans un tamis moléculaire mésoporeux, dans lequel la zéolithe est choisie parmi MFI, MTT, TON, AEF, MWW, FER, BEAU, FAU, et zéolithes MOR, **caractérisé en ce que** la source d'aluminium supplémentaire est choisie parmi les alcoxydes d'aluminium, de préférence l'isopropoxyde d'aluminium.
